# EUROPEAN PATENT SPECIFICATION

(11) **EP 1 529 116 B1**
(45) Date of publication and mention of the grant of the patent: **01.07.2009**
(21) Application number: 03747881.5
(22) Date of filing: 06.08.2003
(51) Int. Cl.: C12Q 1/68, A61K 31/27

(54) **Method for predicting the responsiveness to treatment with rivastigmine based on the ApoE genotyp of dementia patients**
Verfahren zur Vorhersage des Behandlungserfolgs mit Rivastigmine basierend auf einer Bestimmung des ApoE Genotyps eines Demenzkranken
Procédé pour pronostiquer la réponse au rivastigmine se basant sur le genotype ApoE du patient soyant atteint de démence

(30) Priority: 07.08.2002 US 401694 P
(43) Date of publication of application: 11.05.2005
(73) Proprietor: NOVARTIS AG, 4056 Basel (CH); Novartis Pharma GmbH, 1230 Wien (AT)
(72) Inventor: LANE, Roger, Michael, New York, NY 10025-6809 (US); POLYMEROPOULOS, Mihael, Hristos, Potomac, MD 20854 (US)
(74) Representative: Crawley, Patrick Edward
(86) International application number: PCT/EP2003/008719
(87) International publication number: WO 2004/015140

(56) References cited:
- WO-A-95/16791
- US-B1- 6 391 553
- WINBLAD B ET AL: "A 1-year, randomized, placebo-controlled study of donepezil in patients with mild to moderate AD" NEUROLOGY, vol. 57, no. 3, 14 August 2001 (2001-08-14), pages 489-495, XP009023203 ISSN: 0028-3878
- FARLOW MARTIN R ET AL: "Apolipoprotein E genotype and gender influence response to tacrine therapy" ANNALS OF THE NEW YORK ACADEMY OF SCIENCES;APOLIPOPROTEIN E, 1996, pages 101-110, XP009023190 Conference;Chicago, Illinois, USA; October 21-22, 1995, 1996 New York Academy of Sciences {a}, 2 East 63rd Street, New York, New York 10021, USA Series: Annals of the New York Academy of Sciences (ISSN 0077-8923)
- SJOGREN M ET AL: "Tacrine and rate of progression in Alzheimer's disease: Relation to ApoE allele genotype" JOURNAL OF NEURAL TRANSMISSION, vol. 108, no. 4, 2001, pages 451-458, XP002265850 ISSN: 0300-9564
- AERSSENS J ET AL: "APOE genotype: no influence on galantamine treatment efficacy nor on rate of decline in Alzheimer's disease." DEMENTIA AND GERIATRIC COGNITIVE DISORDERS. SWITZERLAND 2001 MAR-APR, vol. 12, no. 2, March 2001 (2001-03), pages 69-77, XP009023193 ISSN: 1420-8008
- NOBILI FLAVIO ET AL: "Brain perfusion follow-up in Alzheimer's patients during treatment with acetylcholinesterase inhibitors" JOURNAL OF NUCLEAR MEDICINE, vol. 43, no. 8, August 2002 (2002-08), pages 983-990, XP001156838 ISSN: 0161-5505
- DATABASE WPI Derwent Publications Ltd., London, GB; AN 2000-264443 XP002265851 "Apolipoprotein E4-specific monoclonal antibody used for immunoassay of APoE does not react with ApoE isoforms 2 and 3" & JP 2000 069963 A (IGAKU SEIBUTSUGAKU KENKYUSHO:KK), 7 March 2000 (2000-03-07)
- STEFANOVA E ET AL: "Cerebral glucose metabolism, cerebrospinal fluid-beta-amyloid1-42 (CSF-Abeta42), tau and apolipoprotein E genotype in long-term rivastigmine and tacrine treated Alzheimer disease (AD) patients." NEUROSCIENCE LETTERS. IRELAND 27 FEB 2003, vol. 338, no. 2, 27 February 2003 (2003-02-27), pages 159-163, XP001156837 ISSN: 0304-3940

## Description

### Background of the Invention

### Field of the Invention

The present invention belongs to the field of pharmacology and medicine.

In particular, this invention relates to the use of genomic analysis to determine responsiveness of patients with dementia to treatment with cholinesterase inhibitors (ChEls).

### Description of the Related Art

Dementia is a heterogeneous and multi-causal group of disease states
characterized by the development of multiple cognitive deficits that include memory impairment and at least one of the following cognitive disturbances: aphasia, apraxia, agnosia or a disturbance in executive functioning. Alzheimer's Disease (AD) or DAT is the leading cause of dementia in the elderly and is the fourth leading cause of death in developed nations (after heart disease, cancer and stroke). Up to 70% of dementia cases are due to AD, with blood vessel disease (stroke, atherosclerosis) being the second most common cause. The frequency of AD among 60-year-olds is about 1%. The incidence doubles approximately every 5 years, becoming 2% at age 65, 4% at 70, 8% at 75, 16% at 80 and 32% at 85. See Forsyth, Phys. Ther., Vol. 78, pp. 1325-1331 (1998); Evans et al., JAMA, Vol. 262, pp. 2551-2556 (1989). It is estimated that as many as two-thirds of those in their 90s suffer from some form of dementia. See Merritt, Textbook of Neurology, 6th Edition, Lea & Febiger, pp. 484-489, Philadelphia, PA (1979); and Gilroy & Meyer, Medical Neurology, MacMillan Publishing Co., pp. 175-179 (1979). AD afflicts an estimated four million human beings in the United States alone at a cost of 100 billion dollars a year. See Schumock, J. Health Syst. Pharm., Vol. 55, No. 52, pp. 17-21 (1998); and Hay & Ernst, Am. J. Public Health, Vol. 77, pp. 1169-1175 (1987).

In addition, the disease is found at much lower levels in the younger age groups, occasionally beginning at about 30 years of age and even rarely in late childhood. See Adams & Victor, Principles of Neurology, pp. 401-407 (1977).

AD is incurable. It leads to death within an average of 8 years after diagnosis, the last 3 of which are typically spent in an institution. Besides memory loss, AD patients show dramatic personality changes, disorientation, declining physical coordination and an inability to care for themselves, impairment of activities of daily living (ADL), progressive cognitive impairment, behavioral disturbance, i.e., wandering, agitation, aggression; and psychiatric signs/symptoms including, but not limited to, psychosis, depression and anxiety. The average rate of major depression, psychosis and agitation in AD is approximately 8%, 20% and 20%, respectively. See Mayeux and Sano, NEJM, Vol. 341, pp. 1670-1679 (1999).

### Genetics of AD

AD is the most common type of dementia that is characterized by progressive cognitive deterioration. See Bayles, Semin. Speech Lang., Vol. 22, No. 4, pp. 251-259 (2001); and Storey et al., Front Biosci., Vol. 7, pp. E155-E184 (2002). Over the last decade, substantial progress has been made in understanding the pathogenesis of AD and several risk factors have been associated with AD including genetic background and age. See Tanzi et al., Neuron., Vol. 32, No. 2, pp. 181-184 (2001) Review; Myers et al., Curr. Opin. Neurol., Vol. 14, No. 4, pp. 433-440 (2001) Review; and Shastry, "Molecular and Cell Biological Aspects of Alzheimer Disease", J. Hum. Genet, Vol. 46, No. 11, pp. 609-618 (2001) Review.

While the exact etiology of AD is unknown, evidence for a genetic contribution comes from several important observations, such as the familial incidence, pedigree analysis, monozygotic and dizygotic twin studies and the association of the disease with Down's syndrome. Studies of the incidence and patterns of transmission in families of patients with AD show that relatives of affected individuals have an increased risk of the development of AD when compared with members of the general population. Concordance rates among co-twins of monozygotic probands with AD are 40-60%, thus suggesting a strong but not absolute genetic influence on the disease. For general review see Baraitser, "The Genetics of Neurological Disorders", 2nd Edition, pp. 85-88 (1990).

Four genes have been linked to AD. These genes are located in chromosomes 1, 14, 19 and 21. Chromosome 21 carries the gene coding for the precursor of β-amyloid, i.e., amyloid precursor protein (APP). This gene was cloned in 1987 after observing that dementia develops in a high proportion of individuals with Down's syndrome (trisomy 21) who survive to adulthood and in whom AD-like pathology was present at autopsy. The subsequent discovery of mutations in the amyloid precursor protein gene in families with early-onset familial AD suggested that overproduction of β-amyloid was associated with some cases of AD.

The presenilin (*PS1*) gene on chromosome 14 was localized in 1992 by using genetic linkage strategies in families with early-onset AD. Approximately 25 different mutations in various areas of the protein have been found in white, Ashkenazi Jewish, Hispanic and Japanese families. Another gene, presenilin 2 (*PS2*), was localized to chromosome 1 after evaluating several large Volga-German kindreds with autosomal dominant early-onset AD. Patients with *PS1* mutations have a characteristic early age of onset, between 35 and 60 years old. *PS2* mutations are found almost exclusively in families of Volga-German heritage. Most patients encountered in the clinic setting are probably not likely candidates for presenilin mutations testing, although *PS1* testing is available commercially. Chromosome 12 may harbor a causative mutation relevant to late-onset AD and is currently under study.

### The Role of Apolipoprotein E (ApoE)

In recent years, research has suggested that ApoE plays a potential role in the pathogenesis of AD. ApoE performs various functions as a protein constituent of plasma lipoproteins, including its role in cholesterol metabolism. It was first identified as a constituent of liver-synthesized very low density lipoproteins (VLDL) that transport triglycerides from the liver to peripheral tissues.

The most important genetic information in patients with late-onset AD comes from the polymorphism analysis of the ApoE gen. See Scarmeas et al., Neurology, Vol. 58, No. 8, pp. 1182-1188 (2002). The ApoE gene contains three major isoforms, ApoE2, ApoE3 and ApoE4, which differ from one another only by single amino acid substitutions. See Rosenberg, "The Molecular and Genetic Basis of Alzheimer's Disease: The End of the Beginning: The 2000 Wartenberg Lecture", Neurology, Vol. 54, No. 11, pp. 2045-2054 (2000).

ApoE is a plasma protein involved in cholesterol transport and is encoded by a gene on chromosome 19. It is synthesized primarily in the liver and is thought to be involved in repair of the nervous systems after injury. ApoE genotype is an important contributor to susceptibility to AD but is found in several other neurodegenerative diseases, such as dementia puglistica. Three common alleles, E2, E3 and E4, correspond to six phenotypes. The ε4 allele has been identified as a risk factor for AD, with the attributable risk estimated to be 45-60%. E4 homozygotes are at a greater risk than E4 heterozygotes. ApoE4 is present in plaques and may facilitate amyloid accumulation in the brain.

The association of the ApoE4 allele with a higher risk and an earlier onset of AD is probably due to its higher affinity for β-amyloid protein compared to other isoforms, which results in the reduced clearance of β-amyloid protein. See Selkoe, "Translating Cell Biology into Therapeutic Advances in Alzheimer's Disease", Nature, Vol. 399, Suppl. 6738, pp. A23-A31 (1999) Review.

ApoE is instrumental in lipoprotein metabolism in several ways. See Mahley et al., J. Lipid Res., Vol. 25, pp. 1277-1294 (1984). It is a recognition site for several cellular lipoprotein receptors, including hepatocyte receptors for chylomicron and VLDL remnants. See Hui et al., J. Biol. Chem., Vol. 259, pp. 860-869 (1984); and Shelburne et al., J. Clin. Invest., Vol. 65, pp. 652-658 (1980).

ApoE-enriched lipoproteins have also been described to have a function in the immune system by inhibiting mitogen- or antigen-stimulated lymphocyte proliferation *in vitro* and *in vivo*. In the ovary, ApoE inhibits androgen production by LH-stimulated cultured theca and interstitial cells. See Dyer et al., J. Biol. Chem., Vol. 263, p. 10965 (1988).

Further substantiation that ApoE- and ApoB-containing lipoproteins are important regulators of lymphocyte function has come from studies of the inhibitory properties of fetal cord blood plasma lipoproteins. See Curtiss et al., J. Immunol., Vol. 133, p. 1379 (1984). In these studies a direct correlation between ApoE and inhibition was established.

There are three major isoforms of ApoE, referred to as ApoE2, ApoE3 and ApoE4 which are products of three alleles at a single gene locus. Three homozygous phenotypes (ApoE2/2, E3/3 and E4/4) and three heterozygous phenotypes (ApoE3/2, E4/3 and E4/2) arise from the expression of any two of the three alleles. The most common phenotype is ApoE3/3 and the most common allele is E3. See Mahley, Science, Vol. 240, pp. 622-630 (1988).

The amino acid sequences of the three types differ only slightly. ApoE4 differs from ApoE3 in that in ApoE4 arginine is substituted for the normally occurring cysteine at amino acid residue 112. The most common form of ApoE2 differs from ApoE3 at residue 158, where cysteine is substituted for the normally occurring arginine. See Mahley (1988), *supra.* ApoE phenotypes and genotypes are well-described and known in the art as described above. The established nomenclature system as well as the phenotypes and genotypes for ApoE, are described in, for example, Zannis et al., J. Lipid Res., Vol. 23, p. 911 et seq. (1982), which is incorporated by reference herein.

Subjects with the ApoE4/4 genotype are as much as eight times as likely to be affected by AD as subjects with the ApoE2/3 or ApoE3/3 genotypes. Further, the average age of onset of AD and the average age of survival is lower for those having one ApoE4 allele, and lowest for those having two ApoE4 alleles (see U.S. Patent No.5,508,167). Thus, a subject's prognosis for AD is more likely to be negative if the subject has an ApoE4 allele and most negative if the subject has more than one ApoE4 allele. The negative prognosis can be viewed in terms of increased likelihood of developing the disease, or of earlier age of onset.

Other ApoE-linked diseases include Type III hyperlipidemia and atherosclerosis. Other evidence indicates that polymorphisms in the ApoE promoter are also associated with increased risk of AD. See Lambert et al., Human Mol. Gen., Vol. 7, p. 533 (1998); and Lambert et al., Human Mol. Gen., Vol. 7, p. 1511 (1998).

Studies have shown that ApoE fragments ranging from 5-22 kDa are present n the post-mortem cerebral spinal fluid from both control patients and patients with AD. The only major band immunoprecipitated by a monoclonal antibody that recognizes the putative toxic domain runs with an apparent molecular weight of about 22 kDa. This fragment likely corresponds to the major ApoE thrombin cleavage product, which has been shown to be protease-resistant. See Weisgraber et al., J. Biol. Chem., Vol. 258, pp. 12348-54 (1983).

Amino acids 130-169 in human ApoE encompass an immunoregulatory domain with both cytostatic and cytotoxic activities against interleukin-2-dependent T cells. This finding is consistent with results of previous studies that implicated residues 141-155 in ApoE's anti-proliferative effect on naive mitogen-activated T cells. See Cardin et al. (1988); and Dyer et al. (1991).

### Treatment of Dementias

Treatment of dementias, including AD is multimodal, tailored to each individual and modified with progression of the disease. Treatment may be divided into three areas: pharmacologic interventions targeting the specific pathophysiology of the dementia, if possible, and pharmacologic agents that ameliorate specific symptoms, such as delusions and sleep abnormalities, and behavioral interventions, which may improve specific symptoms and improve the patient's activities of daily living.

Treatment goals for AD are: (1) *symptomatic* - to improve the degree of cognitive impairment, including attentional deficits and executive dysfunction, behavioral symptoms, and performance of activities of daily living; and/or (2) *disease modifying* - slow the progression of the disease and to therefore maintain independent function for as long as possible.

The only successful pharmacological treatment of cognitive impairment of AD to date has been the ChEI drugs. These include; tacrine, donepezil, rivastigmine and galantamine. See Jacobsen, "Alzheimer's Disease: An Overview of Current and Emerging Therapeutic Strategies", Curr. Top. Med. Chem., Vol. 2, No. 4, pp. 343-352 (2002); and Giacobini, Ann. N.Y. Acad. Sci., Vol. 920, pp. 321-327 (2000) Review.

ChEls used for the treatment of AD are shown in Table 1 below.

**Table 1. ChEIs for the Treatment of AD**

| | |
|---|---|
| 1 | Rivastigmine (EXELON^{™} or ENA 713) |
| 2 | Tacrine (COGNEX^{™}) |
| 3 | Donepezil (ARICEPT^{™}) |
| 4 | Huperzine-A |
| 5 | Metrifonate |
| 6 | Galantamine (REMINYL^{™}) |
| 7 | Physostigmine |
| 8 | Eptastigmine |
| 9 | Ibedenone |
| 10 | Zifrosilone |
| 11 | Quilostigmine |

ChEls are the only known effective class of agents to target the pathophysiology of the disease and work by increasing the acetylcholine levels found to be decreased in the brains of patients with AD. These drugs may inhibit acetylcholinesterase only or may inhibit both acetyl- and butyryl-ChE. Rivastigmine effectively inhibits both acetyl- and butyryl-ChE and this dual inhibition may account for this drugs special properties.

Some of these ChEl drugs have been shown to be efficacious in both improving the extent of the cognitive deficit in patients with AD, as measured, for example, by the Alzheimer's Disease Assessment Scale (ADAS) scores, but also in improving the neuropsychiatric disturbances of patients with AD, such as agitation, wandering, combativeness, apathy and anxiety. In addition, some of the acetylcholinesterase inhibitor (AChEl) drugs are able to slow the process of deterioration and so stabilise the disease process for certain periods of time. The precise mechanism by which these drug produce all the benefits seen in AD is not fully known it seems clear that they do more than just produce a temporary increase in cholinergic neuro-transmission.

In general, ChEI drugs are effective in patients who have dementias with cholinergic deficits, this includes, but is not limited to, AD, dementia with Lewy bodies and Parkinson's disease and dementia. In general, these drugs should be used early in the course of the dementia, especially AD.

The human brain contains two major types of ChE, AChE and butyrylcholinesterase (BuChE). The main role of AChE is to terminate the impulse transmission at cholinergic synapses by rapid hydrolysis of acetylcholin (ACh). See Giacobini, *supra.*

Until recently, AChE has been considered as a main target for the drug design and the relative contribution of BuChE to the regulation of ACh levels has been largely ignored. However, a growing line of evidence indicates that AChE and BuChE both have roles in the regulation of ACh levels and may also be important in development and progression of AD. See Greig et al., Curr. Med. Res. Opin., Vol. 17, No. 3, pp. 159-165 (2001).

While all ChEls share the common effect of AChE inhibition, rivastigmine is a dual inhibitor of both AChE and BuChE and has demonstrated significant and clinically relevant benefits for the patients with AD. See Bar-On et al., "Kinetic and Structural Studies on the Interaction of Cholinesterases with the Anti-Alzheimer Drug Rivastigmine", Biochem., Vol. 41, No. 11, pp. 3555-3564 (2002). In addition, it has been found that patients who do not respond to or can not tolerate donepezil (ARICEPT™) often will respond and/or tolerate treatment, when switched to rivastigmine. See, Auriacombe et al., Current Medical Research Opinion, Vol. 18, pp. 129-138 (2002).

Although the ChEIs have been successful drugs, only a subgroup of AD patients benefits from them. See Bums et al., Dement. Geriatr. Cogn. Disord., Vol. 10, No. 3, pp. 237-244 (1999); and Rosler et al., BMJ, Vol. 318, No. 7184, pp. 633-638 (1999). In general, at least one-third of the patients do not respond to the treatment with ChEIs at all. Thus there is a great need to establish a method to predict ahead of time a patients probable response to this class of drugs in order to avoid exposing patients to a drug regimen if they will not benefit from it. It would be of great clinical value if a patient's genetic profile could be used to provide predictors of response to the ChEI therapy. However, in the past studies of the effect of ApoE genotype on response to ChEIs have delivered conflicting results. ApoE4 phenotype has been shown to be either a negative predictor or no effect on the efficacy of tacrine treatment in patients with AD. See Sjogren et al., J. Neural. Transm. Vol. 108, No. 4, pp. 451-458 (2001); Farlow et al., Ann. N.Y. Acad. Sci., Vol. 802, pp. 101-110 (1996); Farlow et al., Neurology, Vol. 50, No. 3, pp. 669-677 (1998); Rigaud et al., Eur. J. Neurol., Vol. 7, No. 3, pp. 255-258 (2000); Poirier et al., Proc. Natl. Acad. Sci. USA, Vol. 92, No. 26, pp. 12260-12264 (1995); and MacGowan et al., Int. J. Geriatr. Psychiatry, Vol. 13, No. 9, pp. 625-630 (1998).

ApoE4 phenotype has also been shown to have no influence on the efficacy of galantamine treatment in AD. See Aerssens et al., Geriatr. Cogn. Disord., Vol. 12, No. 2, pp. 69-77 (2001); and Wilcock et al., BMJ, Vol. 321, No. 7274, pp. 1445-1449 (2000).

Nobili et al, J. Nucl. Med., vol. 43(8), p. 983-990 (2002) discloses that Apo E genotypes are comparable in stabilised and non-stabilised patients treated with rivastigmine or donepezil.

All of the ChEls can cause side effects, such as nausea, vomiting, diarrhea, insomnia, muscle cramps, fatigue and anorexia. These side effects which may range from mild to severe. In addition, not all patients with AD will show improvement or even a slowing in the rate of deterioration when taking these drugs. Thus there is a need for methods to determine which patients will respond to a ChEI and which patient will develop adverse side effects during treatment.

One of the variables of greatest practical concern to caregiver of patients with dementia is estimating the level of care that the patient will require in the future. By "level of care" is meant the extent and intensity of outside assistance and monitoring the patient will require to remain safe, and to maximize quality of life and health.

The variation of the level of care for patients with dementia is enormous. The expense of the higher levels of care can be very great as well. The level can range from; minimal for patients with very early and mild disease who may still be fully autonomous and living independently, to home care of increasing intensity, to institutionalization at varying levels of personal restriction and intensity of nursing assistance. In more severe cases, in addition to the progressive cognitive decline, severe behavior problems often occur. These behavior problems include, but are not limited to, depression, aggression or repeated wandering behavior and these behavior problems, as well as the concurrent medical problems that frequently develop in these patients, may require extremely expensive inpatient hospitalization on medical and/or psychiatric units.

It is expected that for any given patient suffering from dementia the required level of care will progressively increase over time. The time course for these changes is measured in months to years However, the rate and nature of this progressive increase cannot be accurately predicted due to variations in the disease process itself and most importantly due to variation in the patients response to treatment, especially to treatment with ChEls. Thus, there is a need, in addition to the above, for methods to predict or estimate in the near future, the required level of care a patient will need, for example, in 6 months, 1 year, 3 years or more. Such a prediction would help in planning to maximize the quality and minimize the expense of such care.

### Summary of the Invention

The present invention overcomes the problems described above by providing a method, as defined in the claims, to predict the responsiveness of patients with dementia including, but not limited to, DAT to treatment with ChEI drugs based on a determination of the ApoE genotype.

A further aspect of the invention is a method to determine the responsiveness of an individual with dementia to treatment with Rivastigmine comprising: determining, for each of the two copies of the ApoE gene present in the individual, the nature of the ApoE genotype, wherein if one or both of the two copies of the ApoE gene present in the individual contain the ε4 allele, then the patient would be placed in a good responder group; or if neither of the two copies of the patients ApoE gene contain the ε4 allele then the patient is placed in a poor responder group. In this aspect the dementia is selected from the group consisting of; DAT, vascular dementia, Lewy body dementia, Parkinson's disease dementia, Down Syndrome dementia and mild cognitive impairment.

### Brief Description of the Drawings

### Detailed Description of the Invention

The present invention provides methods to determine which patients with dementia including, but not limited to, AD will be most likely to respond to treatment with rivastigmine with improvement of cognitive functions, improvement in behavioral problems or stabilisation of clinical deterioration in either or both symptom groups. The term "respond to treatment" as used herein is intended to include both a prophylactic or protective effect whereby the symptoms of the disease are either prevented from occurring or prevented from worsening for a period of time and, in addition, an effect whereby symptoms that have already manifested themselves are ameliorated or reduced in intensity, severity or duration.

A further aspect of the invention is a method to determine, ahead of time, how responsive a patient will be to treatment with rivastigmine, comprising determining for the two copies of the ApoE gene present in the patient the nature of the two alleles. If one or both genes carries the ε4 isoform allele then the patient will be placed in a good responder group and would be expected to respond to treatment with ChEIs, including treatment with rivastigmine at doses of 6 mg/day or greater. If the ε4 allele is not present in either copy of the ApoE gene then the patient would be placed in the poor responder group. The determination of the responsiveness of a patient to treatment with ChEIs prior to actual treatment would have value in helping the clinician provide the optimal treatment regimen for a patient and to minimize adverse side effects.

As used herein, the term "treatment with 6 mg/day or more of rivastigmine" shall mean administering to a patient, by any means, the molar equivalent of 6 mg or more of rivastigmine tartrate (EXELON™) during a 24-hour period. Rivastigmine tartrate (EXELON™) is commercially available from Novartis Pharmaceutical Corporation, East Hanover, NJ. The rivastigmine may be administered as a single dose or may be subdivided into any number of separate smaller doses to be administered during the 24-hour period. The rivastigmine may be in any pharmaceutically acceptable form that will allow absorption by the body, including as the salt of a mineral or organic acid and may be combined or formulated with any acceptable pharmaceutical excipients. The administration may be by any route including, but not limited to, oral, as an aqueous solution or by either immediate release or delayed release, or slow release; oral pharmaceutical formulation; parenteral, including by intramuscular, intravenous or intrathecal routes; intranasal; rectal; vaginal or by trans-cutaneous absorption or diffusion, including by transdermal patches including, but not limited to, the TRANS DERMAL SYSTEM PATCH™.

### Diagnostic Features of Dementia

In a patient with clinical findings suggesting any dementia like process, including AD, all other causes of the symptoms should be excluded by history, examination and the appropriate laboratory studies. In certain types of dementia laboratory studies can be very helpful. For example, cerebral spinal fluid (CSF) evaluation for amyloid protein and tau protein can increase the likelihood of a diagnosis of AD, but they are not sufficiently specific to be of routine value in screening or early diagnosis of AD. Improvements in these tests in combination with quantitative magnetic resonance imaging (MRI) may ultimately allow early, specific testing. Presence of the ApoE4 allele makes it very likely that the patient's dementia is produced by AD. The ApoE4 presence is also associated with vascular dementia.

As used herein, the terms "AD and DAT" are taken to mean the same disease and are used interchangeably.

Dementias of all types including, but not limited to, AD result in progressive deterioration in the functioning of the patient and result in steadily worsening behavioral problems that coincide with the deterioration in cognitive functioning and are part of the same disease process. Typical behavioral problems shown by patients with dementia include, but are not limited to, depression, psychosis, delusions, sleep disturbance, wandering, anger outbursts, aggression, agitation, apathy, anxiety, suspiciousness, fearfulness and paranoia. In the final stages of most forms of dementia, including AD, victims are bedridden, lose urinary and bowel control and suffer epileptic attacks. Death is usually due to pneumonia or urinary tract infection.

While a definite diagnosis of, for example, AD requires tissue examination at autopsy or biopsy, the diagnosis can be made with high accuracy by using clinical criteria. The clinical manifestations of AD are fairly characteristic, memory disturbance occurs early in the disease; patients have difficulty learning and remembering new material. Spatial and temporal disorientation also may occur early, with patients becoming lost in familiar surroundings. Aphasia, apraxia and acalculia develop as the disease progresses, and apathy or paranoia may occur. Patients often have delusions of theft and spousal infidelity. Patients may wander, pace, open and close drawers repeatedly, and repeat the same questions. Sleep-wake cycle abnormalities may become evident; for example, a patient may be awake at night but think that it is daytime. Activities of daily living decline throughout the illness. Patients lose the ability to eat and groom themselves and have difficulty dressing. In the terminal stages of the disease, patients exhibit cognitive decline in virtually all intellectual spheres, motor abnormalities become evident and both urinary and fecal incontinence develops.

The essential feature of any dementia is the development of multiple cognitive deficits that include memory impairment and at least one of the following cognitive disturbances: aphasia, apraxia, agnosia or a disturbance in executive functioning. The cognitive deficits must be sufficiently severe to cause impairment in occupational or social functioning and must represent a decline from a previously higher level of functioning.

A diagnosis of a dementia should not be made if the cognitive deficits occur exclusively during the course of a delirium. However, a dementia and a delirium may both be diagnosed if the dementia is present at times when the delirium is not present. Dementia may be etiologically related to a general medical condition, to the persisting effects of substance use (including toxin exposure), or to a combination of these factors.

Memory impairment is required to make the diagnosis of a dementia and is a prominent early symptom (Criterion A1). Individuals with dementia become impaired in their ability to learn new material, or they forget previously learned material. Most individuals with dementia have both forms of memory impairment, although it is sometimes difficult to demonstrate the loss of previously learned material early in the course of the disorder. They may lose valuables like wallets and keys, forget food cooking on the stove, and become lost in unfamiliar neighbourhoods. In advanced stages of dementia, memory impairment is so severe that the person forgets his or her occupation, schooling, birthday, family members and sometimes even name.

Memory may be formally tested by asking the person to register, retain, recall and recognise information. The ability to learn new information may be assessed by asking the individual to learn a list of words. The individual is requested to repeat the words (registration), to recall the information after a delay of several minutes (retention, recall), and to recognise the words from a multiple list (recognition). Individuals with difficulty learning new information are not helped by clues or prompts, e.g., multiple-choice questions, because they did not learn the material initially. In contrast, individuals with primarily retrieval deficits can be helped by clues and prompts because their impairment is in the ability to access their memories. Remote memory may be tested by asking the individual to recall personal information or past material that the individual found of interest, e.g., politics, sports, entertainment. It is also useful to determine (from the individual and informants) the impact of the memory disturbances on the individual's functioning, e.g., ability to work, shop, cook, pay bills, return home without getting lost.

Deterioration of language function (aphasia) may be manifested by difficulty producing the names of individuals and objects (Criterion A2a). The speech of individuals with aphasia may become vague or empty, with long circumlocutory phrases and excessive use of terms of indefinite reference, such as "thing" and "it". Comprehension of spoken and written language and repetition of language may also be compromised. In the advanced stages of dementia, individuals may be mute or have a deteriorated speech pattern
characterized by echolalia, i.e., echoing what is heard; or palilalia, i.e., repeating sounds or words over and over. Language is tested by asking the individual to name objects in the room, e.g., tie, dress, desk, lamp; or body parts, e.g., nose, chin, shoulder; follow commands, e.g., "point at the door and then at the table"; or repeat phrases, e.g., "no ifs, ands or buts".

Individuals with dementia may exhibit apraxia, i.e., impaired ability to execute motor activities despite intact motor abilities, sensory function and comprehension of the required task (Criterion A2b). They will be impaired in their ability to pantomime the use of objects, e.g., combing hair; or to execute known motor acts, e.g., waving goodbye. Apraxia may contribute to deficits in cooking, dressing and drawing. Motor skill disturbances may be tested by asking the individual to execute motor functions, e.g., to show how to brush teeth, to copy intersecting pentagons, to assemble blocks or to arrange sticks in specific designs.

Individuals with dementia may exhibit agnosia, i.e., failure to recognize or identify objects despite intact sensory function (Criterion A2c). For example, the individual may have normal visual acuity but lose the ability to recognize objects, such as chairs or pencils. Eventually they may be unable to recognize family members or even their own reflection in the mirror. Similarly, they may have normal tactile sensation, but be unable to identify objects placed in their hands by touch alone, e.g., a coin or keys.

Disturbances in executive functioning are a common manifestation of dementia (Criterion A2d) and may be related especially to disorders of the frontal lobe or associated subcortical pathways. Executive functioning involves the ability to think abstractly and to plan, initiate, sequence, monitor and stop complex behavior. Impairment in abstract thinking may be manifested by the individual having difficulty coping with novel tasks and avoiding situations that require the processing of new and complex information. The ability to abstract can be formally assessed by asking the person to find similarities or differences between related words. Executive dysfunction is also evident in a reduced ability to shift mental sets, to generate novel verbal or non-verbal information, and to execute serial motor activities.

Tests for executive function include asking the individual to count to 10, recite the alphabet, subtract serial 7s, state as many animals as possible in 1 minute, or draw a continuous line consisting of alternating m's and n's. It is also useful to determine (from the individual and informants) the impact of the disturbances in executive functioning on the individual's daily life, e.g., ability to work, plan activities, budget.

The items in both Criterion A1 (memory impairment) and Criterion A2 (aphasia, apraxia, agnosia or disturbance in executive functioning) must be severe enough to cause significant impairment in social or occupational functioning, e.g., going to school, working, shopping, dressing, bathing, handling finances and other activities of daily living; and must represent a decline from a previous level of functioning (Criterion B). The nature and degree of impairment are variable and often depend on the particular social setting of the individual. The same level of cognitive impairment may significantly impair an individual's ability to perform a complex job, but not a job that is less demanding. Standardized published rating scales that measure physical maintenance, e.g., personal hygiene; intellectual functioning, and the ability to use implements or tools, e.g., telephone, washing machine; can be used to measure the severity of impairment.

Dementia is not diagnosed if these symptoms occur exclusively during the course of a delirium. However, a delirium may be superimposed on a pre-existing dementia, in which case both diagnoses should be given.

### Associated Features and Disorders

### Associated descriptive features and mental disorders

Individuals with dementia may become spatially disoriented and have difficulty with spatial tasks. Visuospatial functioning can be assessed by asking the individual to copy drawings, such as a circle, overlapping pentagons and a cube. Poor judgment and poor insight are common in dementia. Individuals may exhibit little or no awareness of memory loss or other cognitive abnormalities. They may make unrealistic assessments of their abilities and make plans that are not congruent with their deficits and prognosis, e.g., planning to start a new business. They may underestimate the risks involved in activities, e.g., driving. Occasionally, they may harm others by becoming violent. Suicidal behavior may occur, particularly in early stages when the individual is more capable of carrying out a plan of action. Dementia is sometimes accompanied by motor disturbances of gait leading to falls.

Some individuals with dementia show disinhibited behavior, including making inappropriate jokes, neglecting personal hygiene, exhibiting undue familiarity with strangers, or disregarding conventional rules of social conduct. Slurred speech may occur in dementia that is associated with subcortical pathology, such as Parkinson's disease, Huntington's disease and some cases of vascular dementia. The multiple cognitive impairments of dementia are often associated with anxiety, mood and sleep disturbances. Delusions are common, especially those involving themes of persecution, e.g., that misplaced possessions have been stolen. Hallucinations can occur in all sensory modalities, but visual hallucinations are most common. Delirium is frequently superimposed on dementia because the underlying brain disease may increase susceptibility to confusional states that may be produced by medications or other concurrent general medical conditions. Individuals with dementia may be especially vulnerable to physical stressors, e.g., illness or minor surgery; and psychosocial stressors, e.g., going to the hospital, bereavement; which may exacerbate their intellectual deficits and other associated problems.

### Associated laboratory findings

A discussion of associated laboratory findings that are specific to types of dementia is included in the text for each dementia. Invariably there are abnormalities in cognitive and memory functioning, which can be assessed using mental status examinations and neuropsychological testing. Neuroimaging may aid in the differential diagnosis of dementia. Computed tomography (CT) or MRI may reveal cerebral atrophy, focal brain lesions (cortical strokes, tumors, subdural hematomas), hydrocephalus or periventricular ischemic brain injury. Functional imaging, such as positron-emission tomography (PET) or single photon emission computed tomography (SPECT) are not routinely used in the evaluation of dementia, but may provide useful differential diagnostic information, e.g., parietal lobe changes in AD or frontal lobe alterations in frontal lobe degenerations; in individuals without evidence of structural changes on CT or MRI scans.

### Associated physical examination findings and general medical conditions

The associated physical examination findings of dementia depend on the nature, location and stage of progression of the underlying pathology. The most common cause of dementia is AD, followed by vascular disease and then by multiple etiologies. Other causes of dementia include Pick's disease, normal-pressure hydrocephalus, Parkinson's disease, Huntington's disease, traumatic brain injury, brain tumors, anoxia, infectious disorders, e.g., human immunodeficiency virus (HIV), syphilis; prion diseases, e.g., Creutzfeldt-Jakob disease; endocrine conditions, e.g., hypothyroidism, hypercalcemia, hypoglycemia; vitamin deficiencies, e.g., deficiencies of thiamine, niacin, vitamin B12; immune disorders, e.g., polymyalgia rheumatica, systemic lupus erythematosus; hepatic conditions, metabolic conditions, e.g., Kufs' disease, adrenoleukodystrophy, metachromatic leukodystrophy and other storage diseases of adulthood and childhood; and other neurological conditions, e.g., multiple sclerosis.

### Diagnostic Criteria for DAT

A. The development of multiple cognitive deficits manifested by both:
   (1) Memory impairment (impaired ability to learn new information or to recall previously learned information); and
   (2) one (or more) of the following cognitive disturbances:
      (a) aphasia (language disturbance);
      (b) apraxia (impaired ability to carry out motor activities despite intact motor function);
      (c) agnosia (failure to recognize or identify objects despite intact sensory function); and/or
      (d) disturbance in executive functioning, i.e., planning, organizing, sequencing, abstracting.
B. The cognitive deficits in Criteria A1 and A2 each cause significant impairment in social or occupational functioning and represent a significant decline from a previous level of functioning.
C. The course is characterized by gradual onset and continuing cognitive decline.
D. The cognitive deficits in Criteria A1 and A2 are not due to any of the following:
   (1) other central nervous system conditions that cause progressive deficits in memory and cognition, e.g., cerebrovascular disease, Parkinson's disease, Huntington's disease, subdural hematoma, normal-pressure hydrocephalus, brain tumor;
   (2) systemic conditions that are known to cause dementia, e.g., hypothyroidism, vitamin B12 or folic acid deficiency, niacin deficiency, hypercalcemia, neurosyphilis, HIV infection; or
   (3) substance-induced conditions.
E. The deficits do not occur exclusively during the course of a delirium.
F. The disturbance is not better accounted for by another Axis I disorder, e.g., major depressive disorder or schizophrenia.

### Code numbers for DAT (based on type of onset and pre-dominant features)

**With Early Onset** - if onset is at age 65 years or below:
   *290.11 With Delirium*: if delirium is superimposed on the dementia.
   *290.12 With Delusions:* if delusions are the predominant feature.
   *290.13 With Depressed Mood*: if depressed mood (including presentations that meet full symptom criteria for a Major Depressive Episode) is the predominant feature. A separate diagnosis of Mood Disorder Due to a General Medical Condition is not given.
   *290.10 Uncomplicated*: if none of the above predominates in the current clinical presentation.
**With Late Onset** - if onset is after age 65 years:
   *290.3 With Delirium*: if delirium is superimposed on the dementia.
   *290.20 With Delusions:* if delusions are the predominant feature.
   *290.21 With Depressed Mood*: if depressed mood (including presentations that meet full symptom criteria for a Major Depressive Episode) is the predominant feature. A separate diagnosis of Mood Disorder Due to a General Medical Condition is not given.
   *290.0 Uncomplicated:* if none of the above predominates in the current clinical presentation.

### Specify if:

### With Behavioral Disturbance

See "Diagnostic and Statistical Manual of Mental Disorders", 4^{th} Edition (DSM-IV), American Psychiatric Association, Washington, DC (1994).

### The Assessment of Severity of Dementias

The degree of severity of symptoms in dementia can be assessed by a trained clinician by clinical examination. To aid this assessment and to allow a standardized and measurable score a variety of clinical assessment scales may be used. The scales commonly used by clinicians to assess cognition include, but are not limited to, the Mini-Mental Status, the Dementia Symptom Assessment Scale and the ADAS. The PDS - Progressive Deterioration Scale, ADCS-ADL, and DAD assess performance of activities of daily living. The Neuropsychiatric Inventory (NPI) and BEHAVE-AD assess behavior. The CIBIC - PLUS and CGIC are assessments of global functioning.

### The ADAS

For research into AD and potential therapeutic interventions and clinical evaluations one commonly used standardised assessment which can be used to measure specifically the severity of major dysfunctions in cognitive and non-cognitive behaviours characteristic of AD sufferers is the ADAS.

The 21-item ADAS is a performance-based scale which includes 11 items to assess cognitive function, e.g., memory and orientation (ADAS-Cog), and 10 items to assess non-cognitive function, e.g., mood state and behavioral changes (ADAS-Noncog) (Table 2). A score between 0 and 70 is possible on the cognitive part of the scale, where 0 means the patient made no errors at all and 70 means the patient is profoundly demented. In practice, however, a healthy individual will probably score between 5 and 10. The scale therefore clearly spans the full range of cognitive from normal to terminally demented. See Rosen et al., Amer. J. Psych., Vol. 141, pp. 1356-1364 (1984).

**Table 2. Structure of the ADAS**

| **Symptom area** | | **Number of items** | **Number of points** |
|---|---|---|---|
| **Cognitive total** | | **11** | **70** |
| | Memory | 3 | 27 |
| | Orientation | 1 | 8 |
| | Language | 5 | 25 |
| | Praxis | 2 | 10 |
| **Non-cognitive total** | | **10** | **50** |

The 11 cognitive items include spoken language ability, comprehension of spoken language, recall of test instructions, word-finding difficulty in spontaneous speech, following commands, naming objects and fingers, constructional praxis, ideational praxis, orientation, word-recall task and word-recognition task. The word-recall task is administered first and then the following 10 minutes are spent in an open-ended conversation in order to assess the various other aspects of language. The remaining cognitive tasks are then administered.

**Table 3. ADAS Item Characteristics**

| | | **Maximum Potential Score** |
|---|---|---|
| **ADAS-Cog** *(cognitive portion of the ADAS)* | | |
| | Word recall | 10 |
| | Orientation | 8 |
| | Ideational practice | 5 |
| | Drawing | 5 |
| | Commands | 5 |
| | Naming | 5 |
| | Word recognition | 12 |
| | Recall of test instructions | 5 |
| | Expressive language | 5 |
| | Language comprehension | 5 |
| | Word-finding difficulties | 5 |
| | | |
| **ADAS-Noncog** *(non-cognitive portion of the ADAS)* | | |
| | Tremors | 5 |
| | Pacing | 5 |
| | Motor restlessness | 5 |
| | Tearfulness | 5 |
| | Depression | 5 |
| | Delusions | 5 |
| | Hallucinations | 5 |
| | Appetite | 5 |
| | Concentration | 5 |
| | Uncooperativeness | 5 |

### EXAMPLE 1

A pharmacogenetic study of the effect of a patients ApoE ε4 genotype on the response to the ChEI, rivastigmine was conducted. Rivastigmine tartrate is an inhibitor of both AChE and BuChE and is commercially available under the name EXELON™ from Novartis Pharmaceuticals Corporation, East Hanover, NJ. Individuals in the trial were divided into treatment groups depending on the dose they received, namely <6 mg/day of rivastigmine, 6-12 mg/day of rivastigmine and placebo. Each treatment group was analyzed separately. Individuals in each treatment group were then classified as ApoE ε4 carriers or non-carriers based on the genotype data. A significant association (P = 0.0071) between the ApoE ε4 carriers and the greater than 4 point improvement was found in the 6-12 mg group. In contrast, the association of the ApoE ε4 allele with non-deterioration in the study was not significant (P = 0.1172) in the same group. A response to treatment was observed for 65% of the ApoE ε4 carriers, compared to 48.7% of the non-ApoE ε4 carriers when treated with a dose of rivastigmine that is greater than or equal to 6 mg (Table 7).

The data was also analyzed to determine if there were significant differences between the different treatments among the ApoE s4 carriers and non-carriers. Highly significant differences between the three treatment groups were found in the ApoE ε4 carriers (non-deterioration P = 0.00000151, 4-point improvement P = 0.00000175), but in the non-ApoE ε4 carriers (non-deterioration P = 0.1137, 4-point improvement P = 0.7916). Among the non-ApoE ε4 carriers there is a statistically significant difference (P= 0.0434) indicating that the non-ApoE ε4 carriers respond to rivastigmine treatment, but the response rates are not as dramatic as the ApoE ε4 carriers (Table 6). There was no trend (P = 0.7106) in the response with the 4-point improvement criteria for the non-ApoE carriers

The association of the change from baseline ADAS-Cog score with the ApoE ε4 allele is significant (P = 0.0357) in the 6-12 mg group. No significant association was found in the other treatment groups. The average change from baseline in the ADAS-Cog score of the individuals carrying the ApoE ε4 allele is -1.72 compared to 0.335 for the non-ε4 carriers when treated with ≥6 mg/day of rivastigmine. This indicates that the ε4 carriers are better responders to rivastigmine treatment.

### Methods

### Samples

The Phase III clinical trials ENA 713 B352 and ENA 713 B351 are multicenter, double-blind, placebo-controlled, parallel-group study to examine the effect of rivastigmine in outpatients with probable AD. Both trials are conducted over a period of 26 weeks. ENA 713 B351 has 4 treatment arms with comprising of rivastigmine 3 mg/day, rivastigmine 6 mg/day, rivastigmine 9 mg/day or placebo. The initial dose-titration phase (Weeks 1-12) was followed by a fixed-dose phase (Week 13-26), for a total randomized double-blind treatment period of 26 weeks. ENA 713 B352 has 3 treatment arms with comprising of rivastigmine 1-4 mg/day, rivastigmine 6-12 mg/day, and placebo. The initial dose-titration phase (Weeks 1-7) was followed by a fixed-dose phase (Week 8-26), for a total randomized, double-blind treatment period of 26 weeks. The blood samples were handled by the Roskamp Institute, University of South Florida.

### Genotyping

Genotyping for the ApoE polymorphisms were performed by the Roskamp Institute, University of South Florida. The genotyping method used employed one-step PCR as described in; Wenham PR, et al., Lancet. 1991 May 11;337(8750):1158-9. The polymorphisms of ApoE are summarized in Table 4.

**Table 4. ApoE Genotype Classification Based on Amino Acid Change in ApoE Sequence**

| **ApoE Classification** | **Amino Acid Position 112** | **Amino Acid Position 158** |
|---|---|---|
| ε2 | Cys | Cys |
| ε3 | Cys | Arg |
| ε4 | Arg | Arg |

### Statistical Analysis

### 4-point improvement in response

Individuals in the trial were classified as being responders if the final change from their baseline ADAS-COG scores is less than or equal to -4 points. A Fisher's Exact test was used to find an association between ApoE genotype and response.

### Non-deterioration in ADAS-Cog

Individuals in the trial were classified as being responders if the final change from their baseline ADAS-Cog scores is less than or equal to 0 points. A Fisher's Exact test was used to find an association between ApoE genotype and response.

Treatment differences in the ApoE ε4 and non-ApoE ε4 carriers were also analyzed. Treatment differences being measured as the 4-point decrease and the non-deterioration classes. An exact Cochran-Armitage test for trend is used to determine if the numbers of responders increase with dose of rivastigmine among each genotype group.

The trial population was divided into three groups based on the treatment they received. The three groups are placebo, rivastigmine <6 mg/day and rivastigmine ≥6 mg/day. All analyses were conducted within each group separately. The genotype data for the ApoE were re-coded for the presence or absence of the ε4 allele. The re-coded genotype data were used as the categorical variable. The re-coded values are given in Table 5.

**Table 5. Re-Classification of ApoE Genotype**

| **ApoE Genotype** | **Recoded Genotype** |
|---|---|
| ε2 / ε3 | Non-ε4 Carrier |
| ε3 / ε3 | |
| ε2 / ε4 | |
| ε3 / ε4 | ε4 Carrier |
| ε4 / ε4 | |

A non-parametric analysis of variance (ANOVA) model was used for the analysis. Change from baseline in the ADAS-Cog score is used as the dependent variable.

As used herein the terms "ε2, ε3, and ε4" and "E2, E3, and E4" respectively, are used interchangeably.

In addition to the association studies conducted, demographic representation (age, sex and race) in the genotyped population was analyzed to check for any over or under representation of the population in the various genotype and treatment groups, see Table 11. Also, differences, if any, in the demographics between the genotyped and the non-genotyped population were checked, see Table 12.

### Results

The result is highly significant (P = 0.0071) in the 4-point improvement group in the treatment group with a dose of ≥6 mg/day rivastigmine, as can be seen in Table 6. No significant genotype effect was seen for the placebo or rivastigmine <6 mg/day groups as can be seen in Table 6.

**Table 6. Comparison of Treatment Response (as measured by 4 points or greater improvement in the ADAS-Cog score, between carriers and non-carriers of ApoE ε4 allele, among the different treatment groups)**

| | **Placebo** | | **rivastigmine <6 mg/day** | | **rivastigmine ≥6 mg/day** | |
|---|---|---|---|---|---|---|
| **Treatment Response** | No ε4 allele | e4 allele | No ε4 allele | ε4 allele | No ε4 allele | ε4 allele |
| No 4-point improvement | 41 | 69 | 32 | 83 | 37 | 54 |
| 4-point improvement | 3 | 2 | 4 | 12 | 4 | 26 |
| P-value | 0.3691 | | 1 | | 0.0071 | |

In the non-deterioration study, the genotype (ApoE ε4 vs. non-ApoE ε4) does not seem to have a significant effect (P = 0.1172) on response to treatment There are no observed genotype effects in the rivastigmine <6 mg/day and placebo-treated patients as seen in Table 7.

**Table 7. Comparison of Treatment Response (as measured by non-deterioration in the ADAS-Cog score, between carriers and non-carriers of ApoE ε4 allele, among the different treatment groups)**

| | **Placebo** | | **rivastigmine <6 mg/day** | | **rivastigmine ≥6 mg/day** | |
|---|---|---|---|---|---|---|
| **Treatment Response** | No ε4 allele | ε4 allele | No ε4 allele | e4 allele | No ε4 allele | ε4 allele |
| Deterioration | 32 | 52 | 24 | 63 | 21 | 28 |
| Non-deterioration | 12 | 19 | 12 | 32 | 20 | 52 |
| P-value | 1 | | 1 | | 0.1172 | |

From Tables 8 and 9 it is evident that carriers of the ApoE ε4 allele exhibit much higher rates of response, especially when administered at least 6 mg/day of rivastigmine. This is especially true in the non-deterioration criteria. On the other hand, among the non-ApoE ε4 carriers there are no significant differences between the three treatment groups.

A difference in response to treatment as measured by the 4-point improvement in ADAS-Cog scale criteria, among ApoE ε4 carriers was found to be significant (P = 0.00000175) as shown in Table 8.

**Table 8. Comparison of Treatment Response (as measured by 4-point improvement criteria in the ADAS-Cog, between different treatment groups among carriers and non-carriers of ApoE ε4)**

| | **ApoE** ε**4 Carriers** | | | **Non-ApoE** ε**4 Carriers** | | |
|---|---|---|---|---|---|---|
| **Treatment Response** | Placebo | Rivastigmine <6 mg/day | Rivastigmine ≥6 mg/day | Placebo | Rivastigmine <6 mg/day | Rivastigmine ≥6 mg/day |
| No 4-point improvement | 69 | 83 | 54 | 83 | 37 | 54 |
| 4-point improvement | 2 | 12 | 26 | 12 | 4 | 26 |
| P-value | 0.00000175 | | | 0.7916 | | |

A difference in response to treatment as measured by the non-deterioration criteria, among ApoE ε4 carriers was also found to be very significant (P = 0.00000151) as shown in Table 9.

**Table 9. Comparison of Treatment Response (as measured by non-deterioration In the ADAS-Cog score, between different treatment groups among the ApoE ε4 carriers and non-carriers)**

| | | **ApoE** ε**4 Carriers** | | **Non-ApoE** ε**4 Carriers** | | |
|---|---|---|---|---|---|---|
| **Treatment Response** | Placebo | rivastigmine <6 mg/day | rivastigmine ≥6 mg/day | Placebo | rivastigmine <6 mg/day | rivastigmine ≥6 mg/day |
| Deterioration | 52 | 63 | 28 | 32 | 24 | 21 |
| Non-Deterioration | 19 | 12 | 52 | 12 | 12 | 20 |
| P-value | 0.00000151 | | | | 0.1137 | |

The non-parametric ANOVA model yielded a significant result (P = 0.0357) indicating that the ApoE ε4 carriers respond better to rivastigmine treatment compared to non-carriers of the ApoE ε4 allele when treated with a daily dose that is 6 mg/day or higher. The results from the non-parametric ANOVA model are given in Table 10.

**Table 10. Results of the Non-Parametric ANOVA Model (mean of change from baseline in ADAS-Cog score at end of study)**

| **ApoE** ε**4 Carrier** | **Placebo** | **Rivastigmine <6 mg/day** | **Rivastigmine ≥6 mg/day** |
|---|---|---|---|
| ε4 allele | 3.15 | 1.293 | -1.74 |
| No ε4 allele | 4.61 | 1.444 | 0.39 |
| P-value | 0.5844 | 0.4845 | 0.0357 |

As it can be seen from Table 10, carriers of ε4 allele in the ≥6 mg dose group have a reduction in their ADAS-Cog score (-1.74). While for the non-ε4 carriers the change from baseline is minimal (0.39). The effect of genotype is not seen in the lower dose group or in the placebo group, indicating that the genotype effect is dependent on the dosage of rivastigmine.

There are no significant differences between the demographic information between the genotyped and the non-genotyped populations as seen in Table 11. Frequencies of the ApoE ε4 genotypes and their distribution among different treatments are also given in Table 12.

**Table 11. Demographic Information of Genotyped and Non-Genotyped Populations**

| | **Race** | | | | **Sex** | | |
|---|---|---|---|---|---|---|---|
| **ApoE** | White | Black Asian/Oriental | | Others | Male | Female | **Age (SD)** |
| Non-genotyped | 926 | 75 | 4 | 19 | 410 | 614 | 74.4 (8.04) |
| Genotyped | 363 | 8 | 2 | 5 | 174 | 204 | 74.03 (7.6) |

| **Distribution of genotyped and non-genotyped individuals in the various treatment groups:** | | | | | | | |
|---|---|---|---|---|---|---|---|
| **Genotype Status** | | **Placebo** | | **0-6 mg/day** | | **6-12 mg/day** | |
| Non-genotyped | | 293 (28.61%)* | | 275 (26.86%)* | | 456 (44.53%)* | |
| Genotyped | | 116 (30.69%)* | | 134 (35.45%)* | | 128 ((33.87%)* | |

| | | | | | | | |
|---|---|---|---|---|---|---|---|
| *Numbers in parentheses indicate percent of row total. | | | | | | | |

**Table 12. Demographic Information in the Different Genotype Groups**

| | **Race** | | | | **Sex** | | **Mean Age (SD)** | **APOE** ε**4 Classification** |
|---|---|---|---|---|---|---|---|---|
| **APOE** | White | Black | Asian/Oriental | Others | Male | Female | | |
| ε2/ε3 | 15 | 1 | 0 | 0 | 12 | 4 | 76.4 (10.8) | No ε4 (121)* |
| ε3/ε3 | 102 | 2 | 0 | 1 | 52 | 53 | 73.9 (9.0) | |
| ε2/ε4 | 13 | 1 | 0 | 0 | 6 | 8 | 74.4 (7.6) | |
| ε3/ε4 | 169 | 2 | 2 | 2 | 79 | 96 | 74.4 (6.9) | ε4 (246)* |
| ε4/ε4 | 53 | 2 | 0 | 2 | 20 | 37 | 72.5 (5.4) | |

| **Distribution of ApoE** ε**4 carrier status among different treatment groups:** | | | | | | | | |
|---|---|---|---|---|---|---|---|---|
| **Genotype Status** | | **Placebo** | | | **0-6 mg/day*** | | **6-12 mg/day** | |
| ApoE ε4 carriers | | 71 | | | 95 | | 80 | |
| ApoE ε4 non-carriers | | 44 | | | 36 | | 41 | |

| | | | | | | | | |
|---|---|---|---|---|---|---|---|---|
| *Numbers in parentheses indicate the number of individuals in that category. *0 and 6 mg doses not included in this category. | | | | | | | | |

### Methods of Genotyping

ApoE phenotypes and genotypes are well described and known in the art. The established nomenclature system as well as the phenotypes and genotypes for ApoE, are described in, for example, Zannis et al., J. Lipid. Res., Vol. 23, p. 911 et seq. (1982), which is incorporated by reference herein. See U.S. Patent No. 5,508,167.

The step of detecting the presence or absence of ApoE4 or of DNA encoding such isoform (including the number of alleles for ApoE4) may be carried out either directly or indirectly by any suitable means. A variety of techniques are known to those skilled in the art. All generally involve the step of collecting a sample of biological material containing either DNA or ApoE from the subject, and then detecting whether or not the subject possesses ApoE4 or DNA encoding such isoform from that sample. For example, the detecting step may be carried out by collecting an ApoE sample from the subject (for example, from cerebrospinal fluid, or any other fluid or tissue containing ApoE), and then determining the presence or absence of an ApoE4 isoform in the ApoE sample (e.g., by-isoelectric focusing or immunoassay).

The isolation and characterization of ApoE is described, for example, in Rall et al., Methods in Enzymology, Vol. 128, pp. 273-287 (1986); Davignon et al., Arteriosclerosis, Vol. 8, pp. 1-21 (1988); and Warnick et al., Clin. Chem., Vol. 25, pp. 279-284 (1979). Isoelectric focusing is an electrophoretic technique by which the molecules are separated based on their isoelectric points (pI) along a continuous pH gradient. Reference proteins, commercially available (e.g., Sigma Chemical Company, St. Louis, MO), are used to indicate a gradient along which the sample proteins match up according to where their pH matches their pI. In Wamick et al. very-low-density ApoEs are isolated from plasma samples and applied to isoelectric focusing gels and the isoelectric focusing patterns of the ApoE isoforms are obtained. According to the Wamick et al. procedure, pI values of the ApoE isoforms, E2, E3 and E4, were about 5.9, 6.0 and 6.1, respectively, in 8 M urea at 4°C. See also Pagnan et al., J. Lipid. Res., Vol. 18, pp. 613-622 (1977); and Utermann et al., FEBS Lett., Vol. 56, pp. 352-355 (1975).

Various isoelectric focusing-type techniques are also provided in Rall et al., *supra,* including analytical isoelectric focusing, cysteamine treatment, neuraminidase treatment, sodium dodecyl sulfate-polyacrylamide gel electrophoresis, as well as amino acid analysis and nucleic acid sequence analysis, and capillary isoelectric focusing is described in Swartz, Biotechnology, Vol. 12, pp. 408-409 (1994).

In the alternative, the detecting step may be carried out by collecting a biological sample containing DNA from the subject, and then determining the presence or absence of DNA encoding an ApoE4 isoform in the biological sample. Any biological sample which contains the DNA of that subject may be employed, including tissue samples and blood samples, with blood cells being a particularly convenient source. The amino acid sequences and nucleic acid sequences for ApoE2, ApoE3 and ApoE4 are known and described. See, e.g., Paik et al., Proc. Natl. Acad. Sci. USA, Vol. 82, pp. 3445-3449 (1985), incorporated by reference herein, for the nucleic acid sequence of ApoE3 and ApoE4; and Mahley (1988), *supra,* for the amino acid sequence information.

Determining the presence or absence of DNA encoding an ApoE4 isoform may be carried out with an oligonucleotide probe labelled with a suitable detectable group, or by means of an amplification reaction, such as a polymerase chain reaction (PCR) or ligase chain reaction (LCR) (the product of which amplification reaction may then be detected with a labelled oligonucleotide probe or a number of other techniques). Further, the detecting step may include the step of detecting whether the subject is heterozygous or homozygous for the gene encoding an ApoE4 isoform. Numerous different oligonucleotide probe assay formats are known which may be employed to carry out the present invention. See, e.g., U.S. Patent No. 4,302,204 to Wahl et al.; U.S. Patent No. 4,358,535 to Falkow et al.; U.S. Patent No. 4,563,419 to Ranki et al.; and U.S. Patent No. 4,994,373 to Stavrianopoulos et al.

Amplification of a selected, or target, nucleic acid sequence may be carried out by any suitable means. See, generally, Kwoh et al., Am. Biotechnol. Lab., Vol. 8, pp. 14-25 (1990). Examples of suitable amplification techniques include, but are not limited to, PCR, LCR, strand displacement amplification (see, generally, Walker et al., Proc. Natl. Acad. Sci. USA, Vol. 89, pp. 392-396 (1992); and Walker et al., Nuc. Acids Res., Vol. 20, pp. 1691-1696 (1992)); transcription-based amplification (see Kwoh et al., Proc. Natl. Acad. Sci. USA, Vol. 86, pp. 1173-1177 (1989)); self-sustained sequence replication (3SR) (see Guatelli et al., Proc. Natl. Acad. Sci. USA, Vol. 87, pp. 1874-1878 (1990)); the Q.beta. replicase system (see Lizardi et al., BioTechnology, Vol. 6, pp. 1197-1202 (1988)); nucleic acid sequence-based amplification (NASBA) (see Lewis, Genetic Engineering News, Vol. 12, No. 9, p. 1 (1992)); the repair chain reaction (RCR) (see Lewis, *supra*); and boomerang DNA amplification (BDA) (see Lewis, *supra*). PCR is currently preferred.

DNA amplification techniques such as the foregoing can involve the use of a probe, a pair of probes, or two pairs of probes which specifically bind to DNA encoding ApoE4, but do not bind to DNA encoding ApoE2 or ApoE3 under the same hybridization conditions, and which serve as the primer or primers for the amplification of the ApoE4 DNA or a portion thereof in the amplification reaction (likewise, one may use a probe, a pair of probes, or two pairs of probes which specifically bind to DNA encoding ApoE2, but do not bind to DNA encoding ApoE3 or ApoE4 under the same hybridization conditions, and which serve as the primer or primers for the amplification of the ApoE2 DNA or a portion thereof in the amplification reaction; and one may use a probe, a pair of probes, or two pairs of probes which specifically bind to DNA encoding ApoE3, but do not bind to DNA encoding ApoE2 or ApoE4 under the same hybridization conditions, and which serve as the primer or primers for the amplification of the ApoE3 DNA or a portion thereof in the amplification reaction).

In general, an oligonucleotide probe which is used to detect DNA encoding ApoE4 is an oligonucleotide probe which binds to DNA encoding ApoE4, but does not bind to DNA encoding ApoE2 or ApoE3 under the same hybridization conditions. The oligonucleotide probe is labelled with a suitable detectable group, such as those set forth below in connection with antibodies. Likewise, an oligonucleotide probe which is used to detect DNA encoding ApoE2 is an oligonucleotide probe which binds to DNA encoding ApoE2 but does not bind to DNA encoding ApoE3 or ApoE4 under the same hybridization conditions, and an oligonucleotide probe which is used to detect DNA encoding ApoE3 is an oligonucleotide probe which binds to DNA encoding ApoE3 but does not bind to DNA encoding ApoE2 or ApoE4 under the same hybridization conditions.

PCR may be carried out in accordance with known techniques. See, e.g., U.S. Patent Nos. 4,683,195; 4,683,202; 4,800,159; and 4,965,188. In general, PCR involves, first, treating a nucleic acid sample (e.g., in the presence of a heat stable DNA polymerase) with one oligonucleotide primer for each strand of the specific sequence to be detected under hybridizing conditions so that an extension product of each primer is synthesized which is complementary to each nucleic acid strand, with the primers sufficiently complementary to each strand of the specific sequence to hybridize therewith so that the extension product synthesized from each primer, when it is separated from its complement, can serve as a template for synthesis of the extension product of the other primer, and then treating the sample under denaturing conditions to separate the primer extension products from their templates if the sequence or sequences to be detected are present. These steps are cyclically repeated until the desired degree of amplification is obtained. Detection of the amplified sequence may be carried out by adding to the reaction product an oligonucleotide probe capable of hybridizing to the reaction product , the probe carrying a detectable label, and then detecting the label in accordance with known techniques, or by direct visualization on a gel.

When PCR conditions allow for amplification of all ApoE allelic types, the types can be distinguished by hybridization with allelic specific probe, by restriction endonuclease digestion, by electrophoresis on denaturing gradient gels, or other techniques. A PCR protocol for determining the ApoE genotype is described in Wenham et al., The Lancet, Vol. 337, pp. 1158-1159 (1991). Examples of primers effective for amplification and identification of the ApoE isoforms are described therein. Primers specific for the ApoE polymorphic region (whether ApoE4, E3 or E2) can be employed. In Wenham, for example, PCR primers are employed which amplify a 227 bp region of DNA that spans the ApoE polymorphic sites (codons 112 and 158, which contain nucleotides 3745 and 3883). The amplified fragments are then subjected to restriction endonuclease Cfol which provides different restriction fragments from the six possible ApoE genotypes which may be recognizable on an electrophoresis gel (see, also, Hixon et al., J. Lipid Res., Vol. 31, pp. 545-548 (1990); Houlston et al., Hum. Genet., Vol. 83, pp. 364-365 (1989); Wenham et al., Clin. Chem., Vol. 37, pp. 241-244 (1991); and Konrula et al., Vol. 36, pp. 2087-2092 (1990) for additional methods.

For an particularly preferred and improved method for genotyping ApoE polymorphisms by use of a PCR-based assay with simultaneous use of two distinct restriction enzymes. See Zivelin et al., Clin. Chem., Vol. 43, No. 9, pp. 1657-1659 (1997).

LCR is also carried out in accordance with known techniques. See, e.g., Weiss, Science, Vol. 254, p. 1292 (1991). In general, the reaction is carried out with two pairs of oligonucleotide probes: one pair binds to one strand of the sequence to be detected; the other pair binds to the other strand of the sequence to be detected. Each pair together completely encompasses the strand to which it corresponds. The reaction is carried out by, first, denaturing (e.g., separating) the strands of the sequence to be detected, then reacting the strands with the two pairs of oligonucleotide probes in the presence of a heat stable ligase so that each pair of oligonucleotide probes is ligated together, then separating the reaction product, and then cyclically repeating the process until the sequence has been amplified to the desired degree. Detection may then be carried out in like manner as described above with respect to PCR.

It will be readily appreciated that the detecting steps described herein may be carried out directly or indirectly. Thus, for example, if either ApoE2 or ApoE3 is also detected in the subject, then it is determined that the subject is not homozygous for ApoE4; and if both ApoE2 and ApoE3 are detected in the subject, then it is determined that the subject is neither homozygous nor heterozygous for ApoE4. Other means of indirectly determining allelic type could be by measuring polymorphic markers that are linked to ApoE allele, as has been demonstrated for the VNTR (variable number tandem repeats) and the ApoB alleles. See Decorter et al., DNA & Cell Biology, Vol. 9, No. 6, pp. 461-469 (1990).

As an alternative to isoelectric focusing and techniques for allele detection, the step of determining the presence or absence of the ApoE4 isoform in a sample may be carried out by an antibody assay with an antibody which selectively binds to ApoE4, i.e., an antibody which binds to ApoE4 but exhibits essentially no binding to ApoE2 or ApoE3 in the same binding conditions. When one wishes to determine the precise ApoE complement of a patient and whether or not that patient is homozygous or heterozygous for ApoE4, then antibodies which selectively bind to ApoE2 and ApoE3 may also be employed, i.e., an antibody which binds to ApoE2 but exhibits essentially no binding to ApoE3 or ApoE4 in the same binding conditions; an antibody which binds to ApoE3 but exhibits essentially no binding to ApoE2 or ApoE4 in the same binding conditions.

Antibodies used to selectively or specifically bind ApoE2, ApoE3 and ApoE4 can be produced by any suitable technique. For example, monoclonal antibodies may be produced in a hybridoma cell line according to the techniques of Kohler and Milstein, Nature, Vol. 265, pp. 495-497 (1975). ApoE2, ApoE3 or ApoE4 may be obtained from a human patient determined to be homozygous therefore, then purified by the technique described in Rall et al., Methods in Enzymol., Vol. 128, p. 273 (1986), and used as the immunogen for the production of monoclonal or polyclonal antibodies. Purified ApoE isoforms may be produced by recombinant means to express a biologically active isoform, or even an immunogenic fragment thereof may be used as an immunogen. Monoclonal Fab fragments may be produced in Escherichia coli from the known sequences by recombinant techniques known to those skilled in the art. See, e.g., Huse, Science, Vol. 246, pp. 1275-1281 (1989) (recombinant Fab techniques); and Wenham et al., Lancet, Vol. 337, p. 1158 (1991) (ApoE PCR primers). The DNA encoding one subtype of ApoE can be obtained and converted to the other by site-directed mutagenesis. See, e.g., Kunkel et al., Methods in Enzymol., Vol. 154, pp. 367-382 (1987); and Kunkel, U.S. Patent No. 4,873,192.

The term "antibodies" as used herein refers to all types of immunoglobulins, including IgG, IgM, IgA, IgD and IgE. The antibodies may be monoclonal or polyclonal and may be of any species of origin, including, for example, mouse, rat, rabbit, horse or human, or may be chimeric antibodies, and include antibody fragments, such as for example, Fab, F(ab')₂ and Fv fragments, and the corresponding fragments obtained from antibodies other than IgG.

An antibody selectively or specifically binding ApoE or a particular ApoE isoform (ligand) generally refers to a molecule capable of reacting with or otherwise recognizing or binding such a ligand. An antibody has binding affinity for a ligand or is specific for a ligand if the antibody binds or is capable of binding the ligand as measured or determined by standard antibody-antigen or ligand-receptor assays, for example, competitive assays, saturation assays, or standard immunoassays, such as ELISA or RIA. This definition of specificity applies to single heavy and/or light chains, CDRs, fusion proteins or fragments of heavy and/or light chains, that are specific for the ligand if they bind the ligand alone or in combination.

Antibody assays (immunoassays) may, in general, be homogeneous assays or heterogeneous assays. In a homogeneous assay the immunological reaction usually involves the specific antibody, a labelled analyte and the sample of interest. The signal arising from the label is modified, directly or indirectly, upon the binding of the antibody to the labelled analyte. Both the immunological reaction and detection of the extent thereof are carried out in a homogeneous solution. Immunochemical labels which may be employed include free radicals, radioisotopes, fluorescent dyes, enzymes, bacteriophages, co-enzymes and so forth.

In a heterogeneous assay approach, the reagents are usually the specimen, the antibody and a system or means for producing a detectable signal. Similar specimens as described above may be used. The antibody is generally immobilized on a support, such as a bead, plate or slide, and contacted with the specimen suspected of containing the antigen in a liquid phase. The support is then separated from the liquid phase and either the support phase or the liquid phase is examined for a detectable signal employing means for producing such signal. The signal is related to the presence of the analyte in the specimen. Means for producing a detectable signal include the use of radioactive labels, fluorescent labels, enzyme labels and so forth. For example, if the antigen to be detected contains a second binding site, an antibody which binds to that site can be conjugated to a detectable group and added to the liquid phase reaction solution before the separation step. The presence of the detectable group on the solid support indicates the presence of the antigen in the test sample. Examples of suitable immunoassays are the radioimmunoassay, immunofluorescence methods, enzyme-linked immunoassays and the like.

Those skilled in the art will be familiar with numerous specific immunoassay formats and variations thereof which may be useful for carrying out the method disclosed herein. See, generally, Maggio, Enzyme-Immunoassay, CRC Press, Inc., Boca Raton, FL (1980). See, also, U.S. Patent No. 4,727,022 to Skold et al., "Methods for Modulating Ligand-Receptor Interactions and their Application"; U.S. Patent No. 4,659,678 to Forrest et al.; U.S. Patent No. 4,376,110 to David et al.; U.S. Patent No. 4,275,149 to Litman et al.; U.S. Patent No. 4,233,402 to Maggio et al.; and U.S. Patent. No. 4,230,767 to Boguslaski et al.

Antibodies which selectively bind an ApoE isoform, i.e., bind to one of ApoE2, ApoE3 or ApoE4 while showing essentially no binding to the other under the same binding conditions, may be conjugated to a solid support suitable for a diagnostic assay (e.g., beads, plates, slides or wells formed from materials, such as latex or polystyrene) in accordance with known techniques, such as precipitation. Antibodies which bind an ApoE isoform may likewise be conjugated to detectable groups, such as radiolabels, e.g., ³⁵S, ¹²⁵I, ¹³¹I; enzyme labels, e.g., horseradish peroxidase, alkaline phosphatase; and fluorescent labels, e.g., fluorescein; in accordance with known techniques.

Kits for determining the presence or absence of ApoE4, and therefore the expected response to treatment with AchEI drugs will include at least one reagent specific for detecting the presence or absence of ApoE4 and instructions for recommended treatment options based on the ApoE4 status. The kit may optionally include a nucleic acid for detection of the ApoE4 gene. Preferably the kit may comprise at least two different nucleic acids for detection of the ApoE4 gene. The kit may comprise at least one gene specific genotyping oligonucleotide for detecting the presence or absence of the ApoE4 allele. Preferably the kit comprises two gene specific genotyping oligonucleotides. In another embodiment the kit may comprise at least one gene specific genotyping primer composition. The primer composition preferably comprises at least one gene specific genotyping oligonucleotide. Most preferably, the composition comprises at least two sets of allele specific primer pairs. The two allele specific genotyping oligonucleotides may be packaged in separate containers.

The kit may optionally include instructions for isoelectric focusing methods for detecting ApoE4.

Similar diagnostic kits for carrying out antibody assays may be produced in a number of ways. In one embodiment, the diagnostic kit comprises: (a) an antibody which binds ApoE2, ApoE3 or ApoE4 conjugated to a solid support; and (b) a second antibody which binds ApoE2, ApoE3 or ApoE4 conjugated to a detectable group. The reagents may also include ancillary agents such as buffering agents and protein stabilizing agents, e.g., polysaccharides and the like. The diagnostic kit may further include, where necessary, other members of the signal-producing system of which system the detectable group is a member (e.g., enzyme substrates), agents for reducing background interference in a test, control reagents, apparatus for conducting a test and the like. Another test kit comprises: (a) an antibody as above; and (b) a specific binding partner for the antibody conjugated to a detectable group. Ancillary agents as described above may likewise be included. The test kit may be packaged in any suitable manner, typically with all elements in a single container along with a sheet of printed instructions for carrying out the test.

A kit for use in determining treatment strategy for a patient with dementia may comprise (a) an antibody able to recognize and bind to the polypeptide expression product of the ApoE4 gene; (b) a container suitable for containing the antibody and a sample of body fluid from the individual wherein the antibody can contact the ApoE4 polypeptide, if it is present; (c) means to detect the combination of the said antibody with ApoE4 polypeptide; and (d) instructions for use of the kit.

Another kit for use in determining treatment strategy for a patient with dementia comprises (a) a polynucleotide able to recognize and bind to the mRNA expression product of the ApoE4 gene; (b) a container suitable for containing the polynucleotide and a sample of body fluid from the patient wherein the said polynucleotide can contact the ApoE4 mRNA, if it is present; (c) means to detect the combination of the said polynucleotide with the ApoE4 mRNA; and (d) instructions for use of the kit.

A further kit for use in determining treatment strategy for a patient with dementia comprising (a) a polynucleotide able to recognize and bind to some portion of the DNA sequence of the ApoE4 gene; (b) a container suitable for containing the polynucleotide and a sample of body fluid from the individual wherein the polynucleotide can contact the ApoE4 DNA sequence, if it is present; (c) means to detect the combination of the said polynucleotide with the ApoE4 DNA sequence; and (d) instructions for use of the kit.

### Immunochemical methods for detecting ApoE4

The immunochemical assay comprises optionally (but preferably) combining a sample, such as a blood sample collected from the subject with a reducing agent, then contacting the sample to a solid support which specifically binds reactive sulfhydryl groups, then separating the sample from the solid support; and then detecting by immunoassay either: (i) the presence or absence of ApoE in said sample, the presence of ApoE4 in said sample indicating the subject has at least one allele for ApoE4, the absence of ApoE4 in said sample indicating said subject has no alleles for ApoE4; or (ii) the presence or absence of ApoE immobilized on the solid support, the presence of ApoE2 or ApoE3 immobilized on said solid support indicating said subject has one or no alleles for ApoE4, the absence of ApoE2 and ApoE3 immobilized on the solid support indicating said subject has two alleles for ApoE4.

Any sample of biological material containing ApoE may be used, including but not limited to a sample of body fluid. For example, the sample may be collected from blood, blood serum, blood plasma, cerebrospinal fluid or any other fluid or tissue containing ApoE from the subject.

It will be readily appreciated that the detecting steps described herein may be carried out directly or indirectly. Thus, for example, if either ApoE2 or ApoE3 is also detected in the subject, then it is determined that the subject is not homozygous for ApoE4; and if both ApoE2 and ApoE3 are detected in the subject, then it is determined that the subject is neither homozygous nor heterozygous for ApoE4. If either ApoE2 or ApoE3 is present the possibility exists that the individual is heterozygous for ApoE4. Heterozygosity for ApoE4 is also indicated if the amount of ApoE bound to the column is determined to be about twice the amount eluted from the column. Alternatively, to make further distinctions, the assay can be used as an initial screen and combined with another assay capable of distinguishing between ApoE2, ApoE3 and ApoE4, such as an immunoassay, isoelectric focusing or PCR analysis of DNA encoding ApoE2, ApoE3 and ApoE4.

Any solid support which specifically binds reactive sulfhydryl groups may be employed. By specific, it is meant that the solid support will covalently bind sulfhydryl groups in the presence of competing groups, such as amino, hydroxyl and carboxylate. These solid supports include, but are not limited to, solid supports employing tresyl chemistry (see Nilsson, Methods Enzymol., Vol. 63, p. 56 (1984)); activated gels having pyridyl disulfide (see Egoroy et al., Proc. Natl. Acad. Sci. USA, Vol. 72, p. 171 (1975)); or dithio-5-nitrobenzoic acid moieties that produce disulfide linkages, and TNB-thiol (5-thio-2-nitrobenzoic acid) agarose gels (e.g., Pierce product number 20409G, available from Pierce Chemical Co., PO Box 117, Rockford, IL 61105). Preferred solid supports are those represented by the formula R-COCH₂X, wherein R is a solid substrate such as a gel and X is halogen, e.g., Cl, Br, I, preferably I. Particularly preferred is the SULFOLINK.RTM.™ coupling gel available as product numbers 44895G, 20405G, 20401 G, 20402G and 20403G from Pierce, PO Box 117, Rockford, IL 61105.

Any agent capable of reducing the disulfide bond in cysteine residues to the corresponding reactive sulfhydryl groups may be used as the reducing agent . Preferably the reducing agent is a lower molecular weight thiol, more preferably mercaptoethanol, dithiothreitol and mercaptoethylamine. Reduction of the disulfide bonds in cysteines will typically be carried out at slightly alkaline and denaturing conditions, for example, 8 M urea or 6 M guanidine HCl.

Once the sample has been contacted to the solid support, it is separated from the solid support by any known means and, if desired, collected for further testing.

The immunoassay step is carried out by specifically binding ApoE with an antibody which specifically binds to ApoE. The antibodies include all types of immunoglobulins, including IgG, IgM, IgA, IgD and IgE produced by any known suitable method as described above.

An advantage of the foregoing technique is that it is not necessary to employ an antibody which binds specifically to one ApoE isoform without binding to one or more of the other isoforms. However, such isoform-specific antibodies may be employed if desired. Antibodies specific for ApoE are readily made and are known.

Immunoassays may, in general, be homogeneous assays or heterogeneous assays, as described above. As also described above, antibodies which specifically bind ApoE may be conjugated to a solid support suitable for a diagnostic assay (e.g., beads, plates, slides or wells formed from materials, such as latex or polystyrene) in accordance with known techniques, and they may likewise be conjugated to detectable groups.

Diagnostic kits for carrying out the immunochemical assay may be produced in a number of ways. the kit may comprise: (a) a solid support which specifically binds reactive sulfhydryl groups; (b) an antibody which binds to ApoE2, ApoE3 and/or ApoE4; and (c), optionally, a reducing agent. Another diagnostic kit may comprise: (a) an antibody which binds ApoE2, ApoE3 and/or ApoE4 conjugated to a solid support; and (b) a second antibody which binds ApoE2, ApoE3 and/or ApoE4 conjugated to a detectable group.

The reagents may also include ancillary agents, such as buffering agents and protein stabilizing agents, e.g., polysaccharides and the like. The diagnostic kit may further include, where necessary, other members of the signal-producing system of which system the detectable group is a member (e.g., enzyme substrates), agents for reducing background interference in a test, control reagents, apparatus for conducting a test and the like. Alternatively a test kit may comprise: (a) an antibody as above; and (b) a specific binding partner for the antibody conjugated to a detectable group. Ancillary agents as described above may likewise be included. The test kit may be packaged in any suitable manner, typically with all elements in a single container along with a sheet of printed instructions for carrying out the test and interpretation of the results.

In brief, blood is collected and allowed to clot, and serum is removed. An aliquot of serum is placed into a reducing agent (e.g., dithiothreitol or β-mercaptoethanol) to reduce the cysteines, thereby producing reactive sulfhydryl groups. The reduced serum is then incubated with an activated support that reacts specifically and quantitatively with sulfhydryl groups (an example of this support is the SULFOLINK.RTM.™ coupling gel produced by Pierce Inc.). Incubation of reduced sera with this activated support will therefore bind all of the ApoE molecules which contain cysteine. After incubation, the serum containing unbound proteins is separated from the activated support with its bound proteins (either by sedimentation or by centrifugation). The serum is then assayed for the presence or absence of ApoE by an antibody specific for ApoE using an ELISA format, with one of several possible detection schemes, e.g., colorometric. ApoE2 or ApoE3 will quantitatively bind to the activated-support, and no free ApoE will be detected in the serum in the ELISA. ApoE4 will not bind to the activated-support, and will therefore be detected in the ELISA assay. Sera of a homozygote ApoE4 patient (containing only the ApoE4 isoform) will have about the same amount of ApoE in the sera after incubation with the activated-support as before incubation. Sera from a heterozygote ApoE4 patient (containing both ApoE4 and another ApoE isoform) will have approximately half the ApoE detected after incubation with the activated-support as before incubation.

Examples of specific genotype analysis are shown below.

### EXAMPLE 2

Genotype analysis for a patient may be performed using high molecular weight DNA, or alternatively RNA, isolated from 5 mL of whole blood drawn from each patient. The ApoE genotype was determined using an allele-specific primer extension method. Primers labeled D, E, F, G and H were synthesized by Genosys Biotech (The Woodland, Tex.) using primer sequences provided in Main et al., J. Lipid Res., Vol. 32, pp. 183-187 (1991). Reactions were carried out in a volume of 50 µL containing 1 µg of DNA; deoxyadenosine triphosphate, deoxycytidine triphosphate, deoxythymidine triphosphate and deoxyguanosine triphosphate, each 0.2 mmol/L; 10% dimethyl sulfoxide; 12.5 pmol of either primer D, E, F or G; 25 pmol of primer H; and 10 µL of 10 x PCR reaction buffer (Vector Biosystem, Toronto, ONT.). The DNA in the reaction mixture was first denatured for 10 minutes at 96°C and then cooled to 4°C. One unit of Taq polymerase (Vector Biosystem, Toronto, ONT.) was then added to each sample. Each sample was re-heated for 2 minutes at 96°C and subjected to 30 cycles in a thermal cycler with each cycle consisting of a 10-second denaturation at 96°C, 30-second annealing at 58°C and 1-minute extension at 65°C. The reaction products were visualized by electrophoresis of 10 µL of the reaction mixture in a 1% agarose gel containing TPE buffer (0.08 mol/L Tris-phosphate, 0.002 mol/L EDTA, Sigma, St-Louis, MO) and ethidium bromide (0.15 µg/mL) for 1 hour at 67v. The gels were then photographed and the banding profile was compared to known standards.

### EXAMPLE 3

Alternatively, the ApoE phenotype can be determined in a patient using a serum or cerebrospinal fluid sample. Proteins are size separated on a 25 cm SDS polyacrylamide gel (10%) and transferred onto a nitrocellulose filter using a BIORAD® Trans-blot cell and detection of the ApoE protein is performed using a polyclonal antibody raised against human ApoE protein (International Immunology Corp., CA, Dil. 1:2000). To control for antibody specificity, adsorption of the anti-ApoE antibody with purified human ApoE protein (MW 34-36 kDa) is performed see if will specifically block ApoE detection. Molecular weight markers (Rainbowmarkers, Amersham) are run in adjacent wells while visualization of the bands is done with a chemiluminescence detection kit (Amersham, Cat. No. RPN 2100). Quantification of the autoradiographic signals is performed using a MCID image analysis system (Ste-Catherine, ONT.) equipped with the 1 D-gel analysis software (see, Published U.S. Patent Application No. US20020086290A1; U.S. Patent No. 6,391,553.

### Other Methods of ApoE Genotyping Employing General SNP Identification Techniques

ApoE genotyping may also be accomplished by general methods to identify SNPs. Many different techniques can be used to identify and characterize SNPs, including single-strand conformation polymorphism (SSCP) analysis, heteroduplex analysis by denaturing high-performance liquid chromatography (DHPLC), direct DNA sequencing and computational methods. See Shi, Clin. Chem., Vol. 47, pp. 164-172 (2001). Thanks to the wealth of sequence information in public databases, computational tools can be used to identify SNPs *in silico* by aligning independently submitted sequences for a given gene (either cDNA or genomic sequences). Comparison of SNPs obtained experimentally and by *in silico* methods showed that 55% of candidate SNPs found by SNPFinder(http://lpgws.nci.nih.gov:82/perl/snp/snp_cgi.pl) have also been discovered experimentally. See Cox et al., Hum. Mutal., Vol.17, pp. 141-150 (2001). However, these *in silico* methods could only find 27% of true SNPs.

The most common SNP typing methods currently include hybridization, primer extension and cleavage methods. Each of these methods must be connected to an appropriate detection system. Detection technologies include fluorescent polarization (see Chan et al., Genome Res., Vol. 9, pp. 492-499 (1999)); luminometric detection of pyrophosphate release (pyrosequencing) (see Ahmadiian et al., Anal. Biochem., Vol. 280, pp, 103-110 (2000)); fluorescence resonance energy transfer (FRET)-based cleavage assays, DHPLC and mass spectrometry (see Shi, *supra*; and U.S. Patent No. 6,300,076 B1)). Other methods of detecting and characterizing SNPs are those disclosed in U.S. Patent Nos. 6,297,018 B1 and 6,300,063 B1.

In one embodiment, the detection of a polymorphism can be accomplished by means of so called INVADER™ technology (available from Third Wave Technologies Inc. Madison, WI). In this assay, a specific upstream "invader" oligonucleotide and a partially overlapping downstream probe together form a specific structure when bound to complementary DNA template. This structure is recognized and cut at a specific site by the Cleavase enzyme, and this results in the release of the 5' flap of the probe oligonucleotide. This fragment then serves as the "invader" oligonucleotide with respect to synthetic secondary targets and secondary fluorescently-labelled signal probes contained in the reaction mixture. This results in specific cleavage of the secondary signal probes by the Cleavase enzyme. Fluorescence signal is generated when this secondary probe, labeled with dye molecules capable of fluorescence resonance energy transfer, is cleaved. Cleavases have stringent requirements relative to the structure formed by the overlapping DNA sequences or flaps and can, therefore, be used to specifically detect single base pair mismatches immediately upstream of the cleavage site on the downstream DNA strand. See Ryan et al., Molecular Diagnosis, Vol. 4, No 2, pp. 135-144 (1999); and Lyamichev et al., Nature Biotechnol., Vol. 17, pp. 292-296 (1999). See also U.S. Patent Nos. 5,846,717 and 6,001,567.

A composition may contain two or more differently labeled genotyping oligonucleotides for simultaneously probing the identity of nucleotides at two or more polymorphic sites. It is also contemplated that primer compositions may contain two or more sets of allele-specific primer pairs to allow simultaneous targeting and amplification of two or more regions containing a polymorphic site.

ApoE genotyping oligonucleotides of the invention may also be immobilized on or synthesized on a solid surface such as a microchip, bead or glass slide (see, e.g., WO 98/20020 and WO 98/20019). Such immobilized genotyping oligonucleotides may be used in a variety of polymorphism detection assays including, but not limited to, probe hybridization and polymerase extension assays. Immobilized ApoE genotyping oligonucleotides may comprise an ordered array of oligonucleotides designed to rapidly screen a DNA sample for polymorphisms in multiple genes at the same time.

An allele-specific oligonucleotide primer has a 3' terminal nucleotide, or preferably a 3' penultimate nucleotide, that is complementary to only one nucleotide of a particular SNP, thereby acting as a primer for polymerase-mediated extension only if the allele containing that nucleotide is present. Allele-specific oligonucleotide primers hybridizing to either the coding or noncoding strand are contemplated. An ASO primer for detecting ApoE gene polymorphisms could be developed using techniques known to those of skill in the art.

Other genotyping oligonucleotides hybridize to a target region located one to several nucleotides downstream of one of the novel polymorphic sites identified herein. Such oligonucleotides are useful in polymerase-mediated primer extension methods for detecting one of the novel polymorphisms described herein and therefore such genotyping oligonucleotides are referred to herein as "primer-extension oligonucleotides". Preferrably, the 3'-terminus of a primer-extension oligonucleotide is a deoxynucleotide complementary to the nucleotide located immediately adjacent to the polymorphic site.

A kit may comprise at least two genotyping oligonucleotides packaged in separate containers. The kit may also contain other components, such as hybridization buffer (where the oligonucleotides are to be used as a probe) packaged in a separate container. Alternatively, where the oligonucleotides are to be used to amplify a target region, the kit may contain, packaged in separate containers, a polymerase and a reaction buffer optimized for primer extension mediated by the polymerise, such as PCR.

The above described oligonucleotide compositions and kits are useful in methods for genotyping and/or haplotyping the ApoE gene in an individual. As used herein, the terms "ApoE genotype" and "ApoE haplotype" mean the genotype or haplotype containing the nucleotide pair or pairs or the nucleotide(s), respectively, that is present at one or more of the polymorphic sites described herein and may optionally also include the nucleotide pair or nucleotide present at one or more additional polymorphic sites in the ApoE gene. The additional polymorphic sites may be currently known polymorphic sites or sites that are subsequently discovered.

One embodiment of the genotyping method involves isolating from the individual a nucleic acid mixture comprising the two copies of the ApoE gene, or a fragment thereof, that are present in the individual, and determining the identity of the nucleotide pair at one or more of the polymorphic sites in the two copies to assign a ApoE genotype to the individual. As will be readily understood by the skilled artisan, the two "copies" of a gene in an individual may be the same allele or may be different alleles. In a particularly preferred embodiment, the genotyping method comprises determining the identity of the nucleotide pair at each polymorphic site.

Typically, the nucleic acid mixture or protein is isolated from a biological sample including a sample of body fluid, taken from the individual, such as a blood sample or tissue sample. Suitable tissue samples include, but are not limited to; whole blood, semen, CSF, saliva, tears, urine, fecal material, sweat, buccal smears, skin and biopsies of specific organ tissues, such as muscle or nerve tissue and hair. The nucleic acid mixture may be comprised of genomic DNA, mRNA or cDNA and, in the latter two cases, the biological sample must be obtained from an organ in which the ApoE gene is expressed. Furthermore it will be understood by the skilled artisan that mRNA or cDNA preparations would not be used to detect polymorphisms located in introns or in 5' and 3' non-transcribed regions. If an ApoE gene fragment is isolated, it must contain the polymorphic site(s) to be genotyped.

One embodiment of the haplotyping method comprises isolating from the individual
a nucleic acid molecule containing only one of the two copies of the ApoE gene, or a fragment thereof, that is present in the individual and determining in that copy the identity of the nucleotide at one or more of the polymorphic sites in that copy to assign a ApoE haplotype to the individual. The nucleic acid may be isolated using any method capable of separating the two copies of the ApoE gene or fragment, including but not limited to, one of the methods described above for preparing ApoE isogenes, with targeted *in vivo* cloning being the preferred approach.

As will be readily appreciated by those skilled in the art, any individual clone will only provide haplotype information on one of the two ApoE gene copies present in an individual. If haplotype information is desired for the individual's other copy, additional ApoE clones will need to be examined. Typically, at least five clones should be examined to have more than a 90% probability of haplotyping both copies of the ApoE gene in an individual. In a particularly preferred embodiment, the nucleotide at each of polymorphic site is identified.

In a preferred embodiment, a ApoE haplotype pair is determined for an individual by identifying the phased sequence of nucleotides at one or more of the polymorphic sites in each copy of the ApoE gene that is present in the individual. In a particularly preferred embodiment, the haplotyping method comprises identifying the phased sequence of nucleotides at each polymorphic site in each copy of the ApoE gene. When haplotyping both copies of the gene, the identifying step is preferably performed with each copy of the gene being placed in separate containers. However, it is also envisioned that if the two copies are labeled with different tags, or are otherwise separately distinguishable or identifiable, it could be possible in some cases to perform the method in the same container. For example, if first and second copies of the gene are labeled with different first and second fluorescent dyes, respectively, and an allele-specific oligonucleotide labeled with yet a third different fluorescent dye is used to assay the polymorphic site(s), then detecting a combination of the first and third dyes would identify the polymorphism in the first gene copy while detecting a combination of the second and third dyes would identify the polymorphism in the second gene copy.

In both the genotyping and haplotyping methods, the identity of a nucleotide(s) (or nucleotide pair or pairs) at a polymorphic site(s) may be determined by amplifying a target region(s) containing the polymorphic site(s) directly from one or both copies of the ApoE gene, or fragment thereof, and the sequence of the amplified region(s) determined by conventional methods. It will be readily appreciated by the skilled artisan that only one nucleotide will be detected at each polymorphic site in individuals who are homozygous at that site, while two different nucleotides will be detected if the individual is heterozygous for that site. The polymorphism may be identified directly, known as positive-type identification, or by inference, referred to as negative-type identification. For example, where a SNP is known to be guanine and cytosine in a reference population, a site may be positively determined to be either guanine or cytosine for all individual homozygous at that site, or both guanine and cytosine, if the individual is heterozygous at that site. Alternatively, the site may be negatively determined to be not guanine (and thus cytosine/cytosine) or not cytosine (and thus guanine/guanine).

In addition, the identity of the allele(s) present at any of the novel polymorphic sites described herein may be indirectly determined by genotyping a polymorphic site not disclosed herein that is in linkage disequilibrium with the polymorphic site that is of interest. Two sites are said to be in linkage disequilibrium if the presence of a particular variant at one site enhances the predictability of another variant at the second site. See Stevens, Mol. Diag., Vol. 4, pp. 309-317 (1999). Polymorphic sites in linkage disequilibrium with the presently disclosed polymorphic sites may be located in regions of the gene or in other genomic regions not examined herein. Genotyping of a polymorphic site in linkage disequilibrium with the novel polymorphic sites described herein may be performed by, but is not limited to, any of the above-mentioned methods for detecting the identity of the allele at a polymorphic site.

The target region(s) may be amplified using any oligonucleotide-directed amplification method including, but not limited to, PCR (see U.S. Patent No. 4,965,188); LCR (see Barany et al., Proc Natl. Acad. Sci. USA, Vol. 88, pp. 189-193 (1991); and WO 90/01069); and oligonucleotide ligation assay (see Landegren et al., Science, Vol. 241, pp. 1077-1080 (1988)). Oligonucleotides useful as primers or probes in such methods should specifically hybridize to a region of the nucleic acid that contains or is adjacent to the polymorphic site. Typically, the oligonucleotides are between 10 and 35 nucleotides in length and preferably, between 15 and 30 nucleotides in length. Most preferably, the oligonucleotides are 20-25 nucleotides long. The exact length of the oligonucleotide will depend on many factors that are routinely considered and practiced by the skilled artisan.

Other known nucleic acid amplification procedures may be used to amplify the target region including transcription-based amplification systems (see U.S. Patent Nos. 5,130,238 and 5,169,766; EP 329,822; and WO 89/06700); and isothermal methods (see Walker et al., Proc Natl. Acad. Sci. USA, Vol. 89, pp. 392-396 (1992)).

A polymorphism(s) in the target region may also be assayed before or after amplification using one of several hybridization-based methods known in the art. Typically, allele-specific oligonucleotides are utilized in performing such methods. The allele-specific oligonucleotides may be used as differently labeled probe pairs, with one member of the pair showing a perfect match to one variant of a target sequence and the other member showing a perfect match to a different variant. In some embodiments, more than one polymorphic site may be detected at once using a set of allele-specific oligonucleotides or oligonucleotide pairs. Preferably, the members of the set have melting temperatures within 5°C and more preferably within 2°C, of each other when hybridizing to each of the polymorphic sites being detected.

Hybridization of an allele-specific oligonucleotide to a target polynucleotide may be performed with both entities in solution or such hybridization may be performed when either the oligonucleotide or the target polynucleotide is covalently or non-covalently affixed to a solid support. Attachment may be mediated, for example, by antibody-antigen interactions, poly-L-Lys, streptavidin or avidin-biotin, salt bridges, hydrophobic interactions, chemical linkages, UV cross-linking baking, etc. Allele-specific oligonucleotides may be synthesized directly on the solid support or attached to the solid support subsequent to synthesis. Solid-supports suitable for use in detection methods include substrates made of silicon, glass, plastic, paper and the like, which may be formed, for example, into wells (as in 96-well plates), slides, sheets, membranes, fibers, chips, dishes and beads. The solid support may be treated, coated or derivatized to facilitate the immobilization of the allele-specific oligonucleotide or target nucleic acid.

The genotype or haplotype for the ApoE gene of an individual may also be determined by hybridization of a nucleic sample containing one or both copies of the gene to nucleic acid arrays and subarrays, such as described in WO 95/11995. The arrays would contain a battery of allele-specific oligonucleotides representing each of the polymorphic sites to be included in the genotype or haplotype.

The identity of polymorphisms may also be determined using a mismatch detection technique including, but not limited to, the RNase protection method using riboprobes (see Winter et al., Proc Natl. Acad. Sci. USA, Vol. 82, p. 7575 (1985); and Meyers et al., Science, Vol. 230, p. 1242 (1985)) and proteins which recognize nucleotide mismatches, such as the *E*. *coli* mutS protein (see Modrich, Ann. Rev. Genet., Vol. 25, pp. 229-253 (1991)). Alternatively, variant alleles can be identified by SSCP analysis (see Orita et al., Genomics, Vol. 5, pp. 874-879 (1989); and Humphries et al., "Molecular Diagnosis of Genetic Diseases", Elles, Ed., pp. 321-340 (1996)) or denaturing gradient gel electrophoresis (see Wartell et at., Nucl. Acids Res., Vol. 18, pp. 2699-2706 (1990); and Sheffield et al., Proc. Natl. Acad . Sci. USA, Vol. 86, pp. 232-236 (1989)).

A polymerase-mediated primer extension method may also be used to identify the polymorphism(s). Several such methods have been described in the patent and scientific literature and include the "Genetic Bit Analysis" method (WO 92/15712) and the ligase/polymerase mediated genetic bit analysis. See U.S. Patent No. 5,679,524. Related methods are disclosed in WO 91/02087, WO 90/09455, WO 95/17676, U.S. Patent Nos. 5,302,509 and 5,945,283. Extended primers containing a polymorphism may be detected by mass spectrometry as described in U.S. Patent No. 5,605,798. Another primer extension method is allele-specific PCR. See Ruafio et al., Nucl. Acids Res., Vol. 17, p. 8392 (1989); Ruafio et al., Nucl. Acids Res., Vol. 19, pp. 6877-6882 (1991); WO 93/22456; and Turki et al., J. Clin. Invest., Vol. 95, pp. 1635-1641 (1995). In addition, multiple polymorphic sites may be investigated by simultaneously amplifying multiple regions of the nucleic acid using sets of allele-specific primers as described in Wallace et al., WO 89/10414.

In a preferred embodiment, the haplotype frequency data for each ethnogeographic group is examined to determine whether it is consistent with Hardy-Weinberg equilibrium. Hardy-Weinberg equilibrium (see Hartl et al., "Principles of Population Genomics", Sinauer Associates, Sunderland, MA, 3rd Edition (1997)) postulates that the frequency of finding the haplotype pair *H*₁/*H*₂ is equal to *P_{H-W}* (*H*₁/*H*₂) = *2p*(*H*₁) *p* (*H*₂) if *H*₁ ≠ *H*₂ and *P_{H-W}* (*H*₁/*H*₂) = *p* (*H*₁) *p* (*H*₂) if *H*₁ = *H*₂. A statistically significant difference between the observed and expected haplotype frequencies could be due to one or more factors including significant inbreeding in the population group, strong selective pressure on the gene, sampling bias, and/or errors in the genotyping process. If large deviations from Hardy-Weinberg equilibrium are observed in an ethnogeographic group, the number of individuals in that group can be increased to see if the deviation is due to a sampling bias. If a larger sample size does not reduce the difference between observed and expected haplotype pair frequencies, then one may wish to consider haplotyping the individual using a direct haplotyping method, such as, for example, CLASPER SYSTEM™ technology (U.S. Patent No. 5,866,404), SMD or allele-specific long-range PCR. See Michalotos-Beloin et al., Nucl. Acids Res., Vol. 24, pp. 4841-4843 (1996).

In one embodiment of this method for predicting a ApoE haplotype pair, the assigning step involves performing the following analysis. First, each of the possible haplotype pairs is compared to the haplotype pairs in the reference population. Generally, only one of the haplotype pairs in the reference population matches a possible haplotype pair and that pair is assigned to the individual. Occasionally, only one haplotype represented in the reference haplotype pairs is consistent with a possible haplotype pair for an individual, and in such cases the individual is assigned a haplotype pair containing this known haplotype and a new haplotype derived by subtracting the known haplotype from the possible haplotype pair. In rare cases, either no haplotype in the reference population are consistent with the possible haplotype pairs, or alternatively, multiple reference haplotype pairs are consistent with the possible haplotype pairs. In such cases, the individual is preferably haplotyped using a direct molecular haplotyping method, such as, for example, CLASPER SYSTEM™ technology (U.S. Patent No. 5,866,404), SMD or allele-specific long-range PCR. See Michalotos-Beloin et al., *supra*).

### Glossary

**"Antibodies"** as used herein includes polyclonal and monoclonal antibodies, chimeric, single chain and humanized antibodies, as well as Fab fragments, including the products of an Fab or other immunoglobulin expression library.

**"Polynucleotide"** generally refers to any polyribonucleotide (RNA) or polydeoxribonucleotide (DNA), which may be unmodified or modified RNA or DNA. "Polynucleotides" include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term "polynucleotide" also includes DNAs or RNAs containing one or more modified bases and DNAs or RNAs with backbones modified for stability or for other reasons.

The present invention is not to be limited in terms of the particular embodiments described in this application, which are intended as single illustrations of individual aspects of the invention. Many modifications and variations of this invention can be made without departing from its scope, as will be apparent to those skilled in the art.

## Claims

1. An *in vitro* method of determining the responsiveness of an individual with dementia to treatment with Rivastigmine comprising:
a) determining, for each of the two copies of the ApoE gene present in the individual, the nature of the ApoE genotype, wherein
b) if one or both of the two copies of the ApoE gene present in the individual contain the ε4 allele, then the patient would be placed in a good responder group; or
c) if neither of the two copies of the patients ApoE gene contain the ε4 allele then the patient is placed in a poor responder group.

2. The method of Claim 1, wherein the dementia is selected from the group consisting of: DAT (dementia of the Alzheimer's type) vascular dementia, Lewy body dementia, Parkinson's disease dementia, Down Syndrome dementia and mild cognitive impairment.

3. The method of Claim 2, wherein the dementia is DAT.

4. The method of Claim 2, wherein the rivastigmine is used at a dose of 6 mg/day or more.

## Patentansprüche

1. In vitro Verfahren zur Bestimmung des Ansprechverhaltens eines Individuums mit Demenz auf eine Behandlung mit Rivastigmin, das umfasst
a) Bestimmung der Art des ApoE Genotyps für jede der zwei Kopien des ApoE Gens, die im Individuum vorkommen, worin
b) falls eine oder beide der zwei Kopien des ApoE Gens, die im Individuum vorkommen, das ε4 Allel enthalten, der Patient einer guten Respondergruppe zugeordnet wird, oder
c) falls keine der zwei Kopien des ApoE Gens des Patienten das ε4 Allel enthalten, der Patient dann einer schlechten Respondergruppe zugeordnet wird.

2. Verfahren nach Anspruch 1, worin die Demenz aus der Gruppe ausgewählt wird, die besteht aus DAT (Demenz vom Alzheimer Typ), vaskulärer Demenz, Lewy-Körperchen Demenz, Demenz durch Parkinson-Krankheit, Demenz durch Down Syndrom und milder, kognitiver Störung.

3. Verfahren nach Anspruch 2, worin die Demenz DAT ist.

4. Verfahren nach Anspruch 2, worin das Rivastigmin mit einer Dosis von 6 mg/Tag oder mehr angewandt wird.

## Revendications

1. Procédé in vitro pour déterminer la faculté de réponse d'un individu atteint de démence à un traitement avec de la Rivastigmine, comprenant:
a) la détermination, pour chacune des deux copies du gène ApoE présent chez l'individu, de la nature du génotype ApoE, dans laquelle
b) si l'une des deux, ou les deux copies du gène ApoE présent chez l'individu contiennent l'allèle ε4, alors le patient serait placé dans un groupe de bons répondeurs; ou
c) si aucune des deux copies du gène ApoE des patients ne contient l'allèle ε4, alors le patient est placé dans un groupe de mauvais répondeurs.

2. Procédé selon la revendication 1, dans lequel la démence est choisie dans le groupe constitué par: la DAT (démence de type Alzheimer), la démence vasculaire, la démence associée aux corps de Lewy, la démence associée à la maladie de Parkinson, la démence associée au syndrome de Down et le déficit cognitif peu sévère.

3. Procédé selon la revendication 2, dans lequel la démence est la DAT.

4. Procédé selon la revendication 2, dans lequel la rivastigmine est utilisée à une dose de 6 mg/jour ou plus.
